# EUROPÄISCHE PATENTANMELDUNG

(11) **EP 4 478 019 A1**
(43) Veröffentlichungstag der Anmeldung: **18.12.2024**
(21) Anmeldenummer: 23179664.0
(22) Anmeldetag: 16.06.2023
(51) Int. Cl.: G01L 1/02, G01L 1/26, G01L 5/00, A61B 5/00, A61F 13/00

(54) **MOBILE DRUCKMESSEINHEIT ZUR MESSUNG DES DRUCKS EINER MEDIZINISCHEN KOMPRESSIONSBINDE AUF EINE EXTREMITÄT**

(71) Anmelder: Paul Hartmann AG, 89522 Heidenheim (DE)
(72) Erfinder: Tamoué, Ferdinand, 67071 Ludwigshafen (DE); Bilgin, Berksoy, 89231 Neu-Ulm (DE); Fernes, Patrick, 89520 Heidenheim (DE)
(74) Vertreter: Paul Hartmann AG Patents & Licensing

(57) **Zusammenfassung**

Die vorliegende Erfindung betrifft eine mobile Druckmesseinheit zur Messung des Drucks einer medizinischen Kompressionsbinde auf eine Extremität wie z.B. ein Bein. Insbesondere ist die mobile Druckmesseinheit geeignet zur Messung des Drucks während einer Kompressionstherapie. Ferner wird der Einsatz im Bereich des Sports zur Beschleunigung der Regeneration und Steigerung der Leistung mittels Kompression ausgeführt. Weiterer Bestandteil der Erfindung ist ein Verfahren zur Herstellung der mobilen Druckmesseinheit.

## Beschreibung

### Technisches Gebiet der Erfindung

Die vorliegende Erfindung betrifft eine mobile Druckmesseinheit zur Messung des Drucks einer medizinischen Kompressionsbinde auf eine Extremität. Insbesondere ist die mobile Druckmesseinheit geeignet zur Messung des Drucks während einer Kompressionstherapie. Ferner wird der Einsatz im Bereich des Sports zur Beschleunigung der Regeneration und Steigerung der Leistung mittels Kompression ausgeführt.

### Hintergrund der Erfindung

Die Versorgung menschlichen Gewebes erfolgt in erster Linie über das Blut. Dieses zirkuliert in Arterien und Venen. Das Risiko für einen Blutstau im venösen System ist höher als in den Arterien, da Venen einen niedrigeren Blutdruck aufweisen. Um dies zu kompensieren, verfügen Venen über Venenklappen, die den Rückfluss des Blutes entgegen der Schwerkraft gewährleisten. Zusätzlich wird dieser Vorgang bei Bewegung durch Muskelkontraktionen unterstützt (Venen-Muskel-Pumpe).

Durch verschiedene Ursachen können die natürlichen Mechanismen jedoch gestört oder überlastet sein. Risikofaktoren beinhalten Rauchen, Diabetes, höheres Alter, länger andauerndes Sitzen bzw. Stehen sowie Bewegungsmangel.

Tritt ein Venenstau über einen längeren Zeitraum auf, sind krankhafte Veränderungen des Gewebes die Folge. Zunächst werden die Gefäßwände auseinandergedrückt. Es kommt zu einer Flüssigkeitsansammlung (Ödem). Blutzellen verklumpen (Thromboseneigung) und lösen eine Entzündungsreaktion aus. Durch die Entzündung wird die Durchlässigkeit der Gefäßwände erhöht. Blutbestandteile und Zellen verlassen die Venengefäße und gelangen in das umliegende Gewebe, wo es zu einer Funktionsstörung kommt. Gleichzeitig führt eine Mangeldurchblutung zu einer ungenügenden Versorgung. Der langfristig resultierende Zustand wird als chronische venöse Insuffizienz bezeichnet. Im weiteren Verlauf kommt es zu einer Dunkelfärbung der betroffenen Hautareale (Hyperpigmentierung), Hautrötung und der Ausbildung von Ekzemen. Im Spätstadium bilden sich Geschwüre, die schließlich zu einem offenen Bein (Ulcus cruris) führen. Diese Wunde zeigt aufgrund der schlechten Durchblutung und der ausbleibenden Abheilung ein hohes Risiko für nachfolgende Infektionen und damit einhergehenden Komplikationen. Die Lebensqualität der Betroffenen ist erheblich eingeschränkt.

Die Kompressionstherapie ist ein etabliertes medizinisches Verfahren, welches vornehmlich in der Phlebologie (Venenheilkunde) Anwendung findet. Sie steigert bzw. erleichtert den venösen Rückfluss, indem sie von außen Druck auf das Gewebe appliziert, wodurch eine Stauung des venösen Blutes in den Gliedmaßen (vornehmlich den Beinen) reduziert oder im Idealfall behoben wird. Daneben kommt die Kompressionstherapie auch bei Stauungen im Lymphsystem (lymphatische Ödeme) zum Einsatz.

Zur Etablierung und Aufrechterhaltung der notwendigen Kompression werden Textilmaterialien verwendet. Hierbei kann zwischen medizinischen Kompressionsbinden und Kompressionsstrümpfen unterschieden werden. Während Kompressionsstrümpfe in der Regel zur Prävention und Rückfallprophylaxe eingesetzt werden, haben sich Kompressionsbinden beim Einsatz während des akuten Krankheitsgeschehens bewährt. Mit diesen wird ein steiferer Kompressionsdruck erzeugt als mit Kompressionsstrümpfen. Allerdings besteht auch bei Kompressionsstrümpfen durch das Übereinanderziehen von zwei Schichten (Ober- und Unterstrumpf) die Möglichkeit den Kompressionsdruck anzupassen bzw. zu steigern.

Darüber hinaus sind adaptive Kompressionsbandagen erhältlich, deren Weite und Druck über Klettverschlüsse einstellbar sind. Zudem gibt es spezielle Kompressionskleidung für Sportler, die darauf abzielt die Durchblutung zu verbessern und somit die Regeneration zu beschleunigen.

Für die Kompressionstherapie mithilfe von Binden stehen Kompressionsbinden in unterschiedlichen Ausführungen zur Verfügung. Die Wahl der richtigen Kompressionsbinde kann entscheidend für den Therapieerfolg sein. Zunächst einmal unterscheiden sich die unterschiedlichen Produkte hinsichtlich dem sogenannten Ruhe- und Arbeitsdruck. Während der Ruhedruck bereits im Liegen Wirkung entfaltet, entsteht der Arbeitsdruck in Bewegung durch das Zusammenwirken von Muskelarbeit und der Binde als Widerlager (Venen-Muskel-Pumpe).

Damit einhergehend unterscheiden sich die verschiedenen Binden in ihrer Dehnbarkeit. Sogenannte Kurzzugbinden sind nur wenig dehnbar, was in Verbänden mit geringer Elastizität resultiert. Sie kombinieren einen hohen Arbeits- mit einem niedrigen Ruhedruck. Kurzzugbinden kommen hauptsächlich während der ersten Behandlungsphase (Entstauungsphase) zum Einsatz. Im Gegensatz dazu haben Langzugbinden ein hohes Dehnungsvermögen, und resultieren in einem geringen Arbeitsdruck bei gleichzeitig hohem Ruhedruck. Zinkleimbinden wiederum verhärten beim Trocknen, nachdem sie feucht angelegt wurden. Durch das Aushärten entsteht der Kompressionsdruck. Nimmt der Beinumfang ab, mindert sich der Druck entsprechend, was einen häufigen Verbandswechsel erforderlich macht.

Bei sogenannten Mehrkomponentensystemen werden Sets genutzt, die aus zwei bis vier Komponenten bestehen. Es kommen Polster-, Kompressions- und Fixierbinden zum Einsatz, um anatomische Unebenheiten auszugleichen und den idealen Druck einzustellen.

Generell können Binden kohäsiv ausgestaltet sein, so dass sie an sich selbst haften und auf diese Weise das Anlegen und die Fixierung des Druckverbands erleichtert wird. Man unterscheidet ein- und beidseitig kohäsive Binden.

Viele Binden sind sterilisierbar (Dampfsterilisation), was der Anwendung auf bereits bestehenden Wunden zugutekommt.

Unpassende Kompressionsstrümpfe oder unsachgemäße Wickelungen können Hautschäden hervorrufen, die Druckstellen, Einschnürungen oder Nekrosen entstehen lassen. Zudem können sich Nervenschädigungen oder tiefe Beinvenenthrombosen entwickeln. Besonders problematisch sind falsch bzw. zu fest angelegte Kompressionsmaterialien bei bereits bestehender Nervenschädigung, wie sie bei Diabetikern häufig zusammen mit Ulcus cruris vorkommt. In einem solchen Fall spürt der Patient die Schädigung des Gewebes durch falsche Kompression nur eingeschränkt oder gar nicht.

Die Kompressionstherapie ist nur erfolgreich, wenn ein Druck innerhalb eines vorgesehenen Druckintervalls eingestellt und anschließend über eine ausreichende Zeit aufrechterhalten wird. Im medizinischen Alltag wird der Druck während der Bandagierung im Regelfall lediglich geschätzt. Dies führt häufig zu Druckwerten, die außerhalb des optimalen Druckintervalls liegen.

Doch auch wenn der Druck korrekt eingestellt wurde, kann er sich im Folgezeitraum verändern. Gründe hierfür sind ein Nachlassen der Geweberückstellkraft innerhalb der Binde (sogenannte Kurzzugbinden sind hierfür besonders anfällig), eine Abnahme des Gewebevolumens (Rückgang von Schwellung bzw. Ödem), sowie Bewegungen des Patienten, die zu einem Verrutschen der Binde führen können.

Um den korrekten Druck einzustellen und Druckabweichungen festzustellen, wurden einige technische Hilfsmittel vorgeschlagen. So beschreibt die WO2014066077A1 ein System zur Druckmessung in der Kompressionstherapie, welches zusätzlich zu einer Druckmesseinheit einen Haltungssensor zur Erfassung der Körperhaltung des Patienten aufweist. Druck- und Haltungsdaten werden in einer Auswerteeinheit verrechnet. Die Auswerteeinheit wird außerhalb des Kompressionstextils befestigt. Dies schränkt jedoch die Alltagstauglichkeit ein und schränkt den Patienten zusätzlich ein (z.B. beim Schlafen). Zudem muss die Auswerteeinheit kontinuierlich mit Strom versorgt werden, was mit der Zeit ein Aufladen bzw. einen Batteriewechsel nötig macht.

Die WO2016073777A1 beschreibt ein System zur Druckmessung in der Kompressionstherapie, welches ein oder mehrere Netzwerkgeräte ("Data collection/transmitter nodes") benötigt. Nachteil bei diesem Ansatz ist, dass die elektronischen Komponenten ungeschützt sind. Dies gilt nicht nur für Bauteile, die außen an der Kompressionsbinde befestigt werden, sondern ebenso für Materialien, die unterhalb der Binde getragen werden, wo sie dem Kompressionsdruck ausgesetzt sind. Gleichzeitig können die vom Kompressionsdruck in die Haut gedrückten Komponenten für den Patienten - vor allem bei langer Tragedauer - unangenehm sein und die Akzeptanz der Therapie bei den Betroffenen herabsetzen.

Hinzu kommt, dass Kompressionsbinden zwar über eine hohe Atmungsaktivität verfügen, aber dennoch die Wärmeabgabe des Körpers einschränken. Komponenten, die zwischen der Kompressionsbinde und der Haut platziert werden, behindern die Abgabe von Schweiß, welcher elektrisch leitende Ionen enthält. Schweiß in größeren Mengen kann die Funktionsbereitschaft oder Genauigkeit der elektronischen Bauteile herabsetzen. Bei einem bereits vorhandenen Ulcus cruris kann zusätzlich zum Schweiß auch Wundsekret hinzukommen. Eine zusätzliche Belastung für die Bauteile entsteht, wenn Patienten diese durch ihr Körpergewicht - beispielsweise im Schlaf - belasten.

Die US2017100300A1 beschreibt ein System zur Druckmessung während der Gewebekompression beim Sporttraining. Dieses System benötigt eine Sende-Empfangseinheit, die auf eine Stromversorgung angewiesen ist. Die Sende-Empfangseinheit wird mittels einer Kordel und eines Drehverschlusssystems am Bein fixiert. Auch dieser Ansatz vermag die oben genannten Nachteile nicht zu überwinden.

Aufgabe der vorliegenden Erfindung ist die Bereitstellung eines Systems zur Messung und Überwachung des Kompressionsdrucks, das schnell und einfach angelegt werden kann, unempfindlich gegenüber äußeren Einflüssen ist, einen hohen Tragekomfort auch während des Schlafens gewährleistet sowie material- und platzsparend ist.

### Zusammenfassung der Erfindung

Die Erfindung betrifft eine kompakte und mobile Druckmesseinheit zur Messung des Kompressionsdrucks, welches nicht auf eine eigene Stromversorgung angewiesen ist. Teil der Erfindung ist weiterhin ein Verfahren zur Herstellung der mobilen Druckmesseinheit sowie ein Set zur Anwendung in der Kompressionstherapie.

Die mobile Druckmesseinheit ist geeignet zur Messung des Anpressdrucks, welcher von einer medizinischen Kompressionsbinde auf eine Extremität oder das Gewebe eines Patienten ausgeübt wird. Die Druckmesseinheit umfasst die folgenden Komponenten:
a) ein gasdichtes Gehäuse, das eine flexible Kuppel sowie in seinem Inneren einen gasgefüllten Hohlraum aufweist, wobei die Kuppel geeignet ist, an die Haut eines Patienten angelegt zu werden, um den Anpressdruck der über dem Gehäuse angelegten Binde aufzunehmen, und wobei die Kuppel vorzugsweise flach ausgebildet ist,
b) einen ersten Drucksensor, welcher außerhalb des gasgefüllten Hohlraums vorhanden ist und welcher mit der atmosphärischen Luft kommuniziert, zur Messung des Außen-Luftdrucks, wobei der Drucksensor an der Gehäuseaußenseite angebracht ist oder zumindest teilweise in das Gehäuse eingelassen ist,
c) einen zweiten Drucksensor, welcher innerhalb des gasgefüllten Hohlraums vorhanden ist und welcher mit diesem kommuniziert, zur Messung des Innen-Luftdrucks in dem gasgefüllten Hohlraum,
d) eine Leiterplatine, welche in dem gasgefüllten Hohlraum vorhanden ist und einen passiven RFID-Transponder umfasst,
wobei die Leiterplatine mit dem ersten und dem zweiten Drucksensor elektrisch verbunden ist, und wobei unter Einsatz eines RFID-Lesegerätes Druckmessungen durch die Drucksensoren durchgeführt und die so ermittelten Daten kabellos ausgelesen werden können.

Die erfindungsgemäße technische Lösung bietet eine Reihe von Vorteilen. Hierzu gehört das Fehlen von Kabeln außerhalb des Gehäuses der Druckmesseinheit. Der Verzicht auf derartige Kabel erhöht nicht nur den Tragekomfort. Er erleichtert und beschleunigt auch das Anlegen, da keine Kabel durch die Wickelungen der Kompressionsbinde geführt werden müssen und vermeidet die Gefahr, dass Kabel durch die Binde in die Haut eingedrückt werden könnten. In diesem Sinne beinhaltet die Erfindung eine mobile Druckmesseinheit, bei der keine Kabel außerhalb des Gehäuses liegen.

Des Weiteren sind die elektronischen Bauteile durch das sie umgebende Gehäuse weitestgehend von äußeren Einflüssen wie Schweiß, Wundsekret, Kompressionsdruck und Körpergewicht weitestgehend geschützt. Gemäß eine bevorzugten Ausführungsform ist die mobile Druckmesseinheit spritzwassergeschützt und kann dadurch bei Bedarf auch desinfiziert werden. Der Spritzwasserschutz kann realisiert werden durch Verwendung wasserundurchlässiger Materialien für das Gehäuse (z.B. PU) und direkte oder indirekte Abschirmung des ersten Drucksensors durch eine luftdurchlässige, aber wasserundurchlässige Membran (z.B. ePTFE). Sensoren, die neben dem Druck auch die Luftfeuchtigkeit messen können, sind im Allgemeinen widerstandsfähiger gegenüber Wasser als konventionelle Drucksensoren. Besonders hohe Wasserbeständigkeit gewährleisten Sensoren, die zur Bestimmung des Wasserdrucks beim Tauchen eingesetzt werden können. So kann das Sensorelement beispielsweise mittels einer Metallmembran vom Messmedium getrennt und durch diese vor äußeren Einflüssen geschützt sein. Weiterhin besteht die Möglichkeit einen mit Gel gefüllten Drucksensor zu verwenden. Das Gel füllt die Hohlräume im Inneren des Sensors aus und bietet einen gewissen Schutz gegen das Eindringen von Wasser und Partikeln. Derartige Sensoren können als erster Drucksensor in der mobilen Druckmesseinheit verbaut werden. Dies hat zudem den Vorteil einer Unempfindlichkeit gegenüber Schweiß, welcher unterhalb eines Kompressionstextils bzw. unterhalb der mobilen Druckmesseinheit von der Haut abgegeben werden kann.

Durch den Verzicht auf eine eigene Stromversorgung ist die Druckmesseinheit sofort einsatzbereit, ohne dass ein Akku aufgeladen oder Batterien eingesetzt werden müssen. Eine Entladung während des Betriebs ist ausgeschlossen. Gleichzeitig kann die Druckmesseinheit hierdurch sehr kompakt ausgestaltet werden. Auf diese Weise kann das System vollständig unter den Kompressionstextilien angebracht und von diesen in Position gehalten werden, so dass es von außen nicht sichtbar ist.

Darüber hinaus können ausreichende Mengen der Druckmesseinheit platzsparend vorrätig gehalten werden. Da eine Entladung ausgeschlossen ist, ist die Lagerungsdauer nahezu unbeschränkt. Die Herstellung der erfindungsgemäßen Druckmesseinheit ist sehr materialsparend und deswegen kostengünstig und nachhaltig.

Durch die kompakte Bauweise ist die Druckmesseinheit sehr leicht, so dass sie den Träger nicht einschränkt. Da auch die Beweglichkeit erhalten bleibt, kann die mobile Druckmesseinheit zusammen mit Sportkompressionskleidung getragen werden, und zwar sowohl während der körperlichen Aktivität als auch danach, um die Regeneration der Muskulatur durch gezielt eingestellte Kompression zu beschleunigen.

### Detaillierte Beschreibung der Erfindung

Der Begriff "mobil" meint im Kontext der vorliegenden Erfindung, dass die Druckmesseinheit ortsunabhängig eingesetzt und dabei mehrere Tage am Stück getragen werden kann. Die Mobilität des Trägers bleibt erhalten.

"Druckmesseinheit" meint eine Vorrichtung, die in der Lage ist, Kompressionsdruck im Rahmen einer Kompressionstherapie zu messen. Somit ist die Druckmesseinheit eine Kompressionsdruckmesseinheit.

Unter dem Begriff "Anpressdruck" wird im weitesten Sinn derjenige Druck verstanden, welchen das Kompressionstextil (z.B. die medizinische Binde) auf das menschliche Gewebe ausübt. Im Kontext der vorliegenden Erfindung kann unter "Anpressdruck" derjenige Druck gemeint sein, mit welchem das Kompressionstextil die Druckmesseinheit an die Haut des Trägers anpresst und der von der Druckmesseinheit erfasst und quantifiziert wird.

Der Begriff "Gewebe" meint insbesondere solches menschliches Gewebe, das bei einem sogenannten Venenstau zur Bildung von Ödemen neigt. Hierbei handelt es sich in erster Linie um das Bindegewebe der Extremitäten, wobei die Beine häufiger betroffen sind als die Arme. Im Kontext der Anwendung zur beschleunigten Regeneration wird unter "Gewebe" auch das Muskelgewebe verstanden.

Unter "Binde" wird eine Kompressionsbinde verstanden. Verschiedene Arten von Kompressionsbinden werden in diesem Dokument aufgeführt und erläutert.

Der Ausdruck "gasdicht" bezieht sich auf das Gehäuse der Druckmesseinheit und meint, dass das Gas, welches sich im Inneren des Gehäuses befindet, unter dem bei Kompressionstherapie anwendbaren Maximaldruck (etwa 80 mmHg) nicht entweicht. Bei dem Gas kann es sich um ein Gasgemisch wie beispielsweise Luft handeln.

"Flexibel" bedeutet, dass ein Material unter Einwirkung einer mechanischen Kraft (z.B. ein Druck von bis zu 200 mmHg) reversibel verformt wird, ohne, dass es zu einer Dehnung oder Stauchung kommt, und wobei das besagte Material nach Beendigung der Krafteinwirkung wieder seine ursprüngliche Form einnimmt.

"Elektrisch verbunden" meint, dass sich ein oder mehrere elektrische Leiter zwischen den verbundenen Elementen befinden, so dass Elektronen durch den Leiter von einem Element zum anderen gelangen können. Ein solcher Leiter kann auch ein Halbleiter sein. Zwei Komponenten, die miteinander elektrisch verbunden sind, können entweder direkt gesteckt sein (z.B. kann ein Drucksensor direkt auf eine Leiterplatine gesteckt sein) oder über Kabel miteinander verbunden sein.

"RFID" steht für "radio-frequency identification". Dieses Verfahren ermöglicht kontaktlosen Datenaustausch zwischen einem RFID-Transponder und einem RFID-Schreib-/Lesegerät. Hierzu baut das RFID-Schreib-/Lesegerät ein magnetisches oder elektromagnetisches Feld auf, welches an der passiven Antenne des RFID-Transponders einen elektrischen Strom erzeugt.

"NFC" steht für "near field communication". Hierbei handelt es sich, um einen spezifischen auf "RFID" basierenden Standard, welcher in der Regel bei einer Frequenz von 13,56 MHz arbeitet und eine kontaktlose Übertragung von Daten auf Distanzen von bis zu etwa zehn Zentimetern ermöglicht.

"Gehäuse" bezieht sich auf denjenigen Teil der Druckmesseinheit, in dessen Innerem sich ein gasgefüllter Hohlraum, eine Leiterplatine und ein Drucksensor befinden. Weitere Komponenten können im Hohlraum oder in den Wänden des Gehäuses vorhanden sein. Die Wände des Gehäuses umfassen zumindest eine flexible Kuppel und optional auch weitere Teile wie z.B. eine Basis.

"Gasdicht" bedeutet, dass bei einer Krafteinwirkung mittels Druck von bis zu 80 mmHg, besser 150 mmHg, am besten 200 mmHg keine nennenswerten Stoffmengen an Gas aus dem Hohlraum des Gehäuses entweichen. Ein minimaler Gasaustritt - z.B. im Bereich von einigen Nanomol - kann jedoch vorkommen und bleibt für die Messgenauigkeit unerheblich.

"Eingebettet" bedeutet, dass eine Komponente teilweise oder vollständig in eine Trägersubstanz eingelassen ist. Eine Einbettung ist eine dauerhafte und irreversible Verbindung, die der eingebetteten Komponente größtmöglichen Schutz gewährleistet und sich positiv auf Lebensdauer und Lagerungsfähigkeit auswirkt.

Soweit auf anatomische oder morphologische Begriffe Bezug genommen wird, sind in erster Linie die entsprechenden Teile des menschlichen Körpers (z.B. Gliedmaßen) gemeint.

Die hier verwendeten Shore-Werte beziehen sich auf die Shore-A-Skala gemessen nach ISO 7619-1.

Die flexible Kuppel kann beispielsweise die Form einer Halbkugel haben. Abwandlungen der Halbkugelform sind möglich (z.B. Bogenform). Die flexible Kuppel kann auch als geometrische Kuppel ausgestaltet sein und weist in diesem Fall Ecken auf. Die Kuppel hat eine dreidimensionale Form. Wenn die Kuppel auf einer Grundfläche steht, sollte das Zentrum der Kuppel ihren höchsten Punkt (gemessen von der Grundfläche aus) bilden.

Die flexible Kuppel besteht aus einem Material, das geeignet ist, an die Haut eines Benutzers angelegt zu werden, also aus einem hautkompatiblen Material. Alternativ besteht die Kuppel aus zwei oder mehr solcher Materialen, die als Gemisch oder als distinkte miteinander verbundene Schichten vorliegen können. Hautkompatibel bedeutet, dass keine giftigen, scharfkantigen oder besonders rauen Materialien verwendet werden dürfen. Ebenso wenig darf das Material mit der Haut reagieren oder auf diese abfärben. Weiterhin darf das Material seine Beschaffenheit nicht verändern, wenn es Körperwärme ausgesetzt wird. Zudem muss das Material der Kuppel gasdicht sein und darf sich bei Druckkräften von bis zu etwa 80 mmHg nicht plastisch verformen. Gleichzeitig soll die Flexibilität der Kuppel eine reversible Verformung unter Druck ermöglichen. Da die Kuppel flexibel ist, passt sie sich den Körperkonturen an. Gleichzeitig findet durch die Kuppelform eine gute Druckverteilung statt, so dass die Druckmesseinheit nicht in die Haut des Trägers gedrückt wird. Dies ist besonders wichtig, da während der Nutzung die mobile Druckmesseinheit durch das Kompressionstextil in Richtung des Körpergewebes gedrückt wird.

Empfohlen werden synthetische Materialien, die die oben genannten Bedingungen erfüllen, da diese innert gegenüber chemischen Einflüssen sind und auf einfache Weise gereinigt werden können. Geeignet sind z.B. bestimmte Kunststoffe einschließlich Weich-PVC, Silikonen (Polyorganosilixanen), Elastomeren und Polyurethanen (PU). Bevorzugt handelt es sich um ein Silikon oder ein thermoplastisches Elastomer. Besonders bevorzugt enthält die flexible Kuppel ein Polyurethan oder besteht aus diesem. Ganz besonders bevorzugt handelt es sich um thermoplastisches Polyurethan (TPU). Gummimaterialien können ebenfalls verwendet werden, solange sie eine ausreichende Zähigkeit aufweisen. Kautschuk-basierte Stoffe sind technisch geeignet, wobei von Materialien, die Kontaktallergien hervorrufen können (z.B. Latex) abgeraten wird. Empfohlen werden nicht-allergene Kautschuk-basierte Materialien wie Neopren. Ein weiteres in Frage kommendes Material natürlicher Herkunft ist Leder. Kombinationen von verschiedenen Materialien sind möglich. So kann das Leder innenseitig mit einer Lage aus z.B. Butylkautschuk kombiniert werden. In diesem Fall kommt die Kautschuk-Verbindung nicht mit der Haut in Berührung und kann keine allergische Reaktion hervorrufen.

Der erste und der zweite Drucksensor können baugleich oder unterschiedlich zu einander sein. Beispielsweise kann der erste Drucksensor zur Messung des Außen-Luftdrucks spritzwassergeschützt oder wasserbeständig sein und der zweite Drucksensor innerhalb des Gehäuses ohne Wasserschutz auskommen. Zusammen mit der Leiterplatine dienen die Drucksensoren dazu die Druckdifferenz zwischen dem Gehäuseinneren und dem Außen-Luftdruck zu bestimmen. Es wird empfohlen zwei Sensoren zu verwenden, welche über dieselbe oder eine vergleichbare Messgenauigkeit verfügen, um Abweichungen bei der Bestimmung der Druckdifferenz zu vermeiden. Die Drucksensoren können als barometrische Drucksensoren ausgestaltet sein. Sie können als kapazitive oder resistive Sensoren vorliegen. Bei dem ersten und / oder zweiten Drucksensor kann es sich um einen Sensor des folgenden Typs handeln: piezoresistiver Drucksensor, frequenzanaloger Drucksensor, kapazitiver Drucksensor, Drucksensor mit Hallelement, induktiver Drucksensor, Silizium-Drucksensor, DMS-Drucksensor. Die Drucksensoren können über einen oder zwei Ladungsverstärker verfügen oder mit solchen gekoppelt sein. Bei Verwendung von piezoresistiven Drucksensoren ist die Verwendung mindestens eines Ladungsverstärkers empfohlen. Wenn beide Drucksensoren piezoresistiv sind, können zwei Ladungsverstärker (pro Sensor ein Ladungsverstärker) verbaut werden. Piezoresistiven Drucksensoren enthalten für gewöhnlich ein piezoresistives Drucksensorelement. Ein solches Element kann Silizium enthalten oder daraus bestehen. Bei Verwendung von Drucksensoren mit Hallelement und Digitalausgang, können bipolare oder unipolare Drucksensoren mit Hallelement eingesetzt werden. Optional können der erste und / oder zweite Drucksensor optional über einen Druckmessumformer verfügen.

Der erste Drucksensor kommuniziert mit der atmosphärischen Luft. D.h. er ist derart positioniert, dass er den Umgebungsluftdruck messen kann. Dazu kann der erste Drucksensor auch teilweise außerhalb des Gehäuses liegen oder in die Gehäusewand eingelassen sein.

Der zweite Drucksensor befindet sich im Inneren des Gehäuses. Vorzugsweise ist er auf die Leiterplatine aufgesteckt und somit im Gehäuseinneren fixiert. Alternativ kann der zweite Drucksensor an einer der Innenwände des Gehäuses befestigt sein und die Verbindung zur Leiterplatine über ein Kabel hergestellt werden.

Empfohlen werden Drucksensoren, die mit einer geringen Stromstärke und einer geringen Spannung arbeiten. Eine geringe Stromstärke kann beispielsweise 1 - 10 µA während der Messung sein. Eine geringe Spannung kann beispielsweise 1 - 4 V während der Messung sein, bevorzugt 1,2 - 3,6 V. Bei Sensoren, die unter Stromversorgung in regelmäßigen Abständen Daten generieren, beziehen sich die zuletzt genannten Werte auf eine Messrate von einer Messung pro Sekunde (1 Hz). Die Drucksensoren können mit einer Temperaturkompensation ausgestattet sein, die Verzerrungen der Messwerte durch Temperaturschwankungen verhindert. Die Temperaturkompensation kann über einen Temperatursensor verfügen. Weiterhin kann die Temperaturkompensation über eine Entkopplungseinheit verfügen oder als solche vorliegen. Bei der Entkopplungseinheit wird die reaktive Einheit des Sensors - bei piezoelektrischen Sensoren also das Piezoelement - auf ein separates Lager gebaut, welches Störgrößen wie durch Temperaturschwankungen hervorgerufene Materialspannungen, weitestgehend verhindert. Drucksensoren, die über eine digitale Schnittstelle verfügen, sind bevorzugt, da hierdurch auf einen zusätzlichen Analog-Digital-Wandler verzichtet werden kann. Stattdessen findet die Digital-Analog-Umwandlung innerhalb des Drucksensors innerhalb einer Auswerteschaltung, auch bekannt als applicationspecific integrated circuit (ASIC), statt. Die reaktive Einheit kann innerhalb eines Micro-Electro-Mechanical-System (MEMS) vorliegen. Das MEMS kann über Bonddrähte mit dem ASIC verbunden werden. Die reaktive Einheit, die ASIC und die Bonddrähte können in einer Hülle und / oder auf einem Sockel untergebracht sein. Bei Drucksensoren mit Digitalschnittstelle wird die Verwendung einer Halbleiterhülle bzw. eines Halbleitersockels empfohlen. Dabei muss nicht die gesamte Hülle aus Halbleitermaterial bestehen. Bei Digitalschnittstellen kann der Sockel als Land Grid Array (LGA), als Pin Grid Array (PGA) oder Ball Grid Array (BGA) ausgestaltet sein. Die Kontakte des Sockels können federnd sein, um mechanische Störeinwirkungen zu minimieren.

Grundsätzlich können alle hier beschriebenen Sensorarten mit einer digitalen Schnittstelle ausgestattet werden. Alternativ kann auch eine lineare Schnittstelle verwendet werden. Gemäß einer besonders bevorzugten Ausführungsform der Erfindung handelt es sich bei dem ersten und / oder dem zweiten Drucksensor um einen digitalen, kalibrierten, linearisierten und temperaturkompensierten Drucksensor.

Die Drucksensoren können über einen Pufferspeicher verfügen, welcher Messwerte zwischenspeichern kann. Der Pufferspeicher kann beispielsweise so ausgestaltet sein, dass 3 - 40 Messwerte gespeichert werden können, bevor sie über die Schnittstelle ausgegeben werden. Der Pufferspeicher hat den Vorteil, dass Messwerte, die schneller generiert als ausgelesen werden, nicht sofort verloren gehen.

Die Leiterplatine kann als printed circuit board (PCB) bzw. printed circuit board assembly (PCBA) vorliegen. Vorzugsweise ist die Leiterplatine flexibel, um sich äußeren Krafteinwirkungen anpassen zu können ohne zu brechen. Zu diesem Zweckkann die Leiterplatine Polyimid enthalten oder daraus gefertigt sein. Vorzugsweise handelt es sich um eine Leiterplatine aus beidseitig kupferbeschichtetem Polyimid, was eine besonders ausgeprägte Flexibilität bei gleichzeitig hoher mechanischer Belastbarkeit garantiert. Der erste und der zweite Drucksensor können direkt auf der Leiterplatine aufgesteckt sein oder mit dieser durch Kabel verbunden sein. Die Leiterplatine umfasst einen RFID-Transponder oder ist mit einem solchen verbunden. Die Leiterplatine kann direkt oder indirekt mit einem RFID-Transponder verbunden sein. Direkt meint in diesem Fall, dass der RFID-Transponder auf die Leiterplatine gesteckt oder in die Leiterplatine integriert bzw. eingebettet vorliegt (die Leiterplatine beinhaltet den RFID-Transponder mitsamt Antenne und RFID-Chip). Indirekt meint, dass der RFID-Transponder mithilfe eines oder mehrerer Kabel an die Leiterplatine angeschlossen ist. Demgemäß kann die mobile Druckmesseinheit gemäß der vorliegenden Erfindung im Gehäuse einen RFID-Transponder umfassen, welcher vorzugsweise direkt oder indirekt mit der Leiterplatine verbunden ist.

Der RFID-Transponder beinhaltet eine Antenne und einen RFID-Chip. Bei dem Transponder kann es sich um einen Read and Write-Transponder handeln. Die zugehörige Speichergröße kann beispielswese 32 byte bis 512 kbyte betragen. Vorzugsweise beträgt die Speichergröße 2 kbyte, 4 kbyte, 8 kbyte, 16 kbyte, 32 kbyte, 64 kbyte oder 128 kbyte. Ein Teil des Speichers kann hierbei auch als Read-Only-Speicher ausgestaltet sein, um ein unerwünschtes Überschreiben von Daten zu vermeiden. Darüber hinaus ist es möglich einen zusätzlichen separaten Read-Only-Speicher in der oben angegebenen Größe einzubauen. Wenn ein Read-Only-Speicher verwendet wird, sollte dieser als permanenter Speicher ausgestaltet sein. Der RFID-Chip kann ein NFC-Chip sein. Der Chip kann als Mikrocontroller vorliegen oder Teil eines solchen sein. Es können auch weitere Komponenten enthalten sein. Die Antenne beinhaltet in der Regel eine Spule und einen Kondensator oder ist mit diesen Einheiten verbunden. Ferner muss die Antenne RFID-fähig sein und somit also in der Lage sein, mit der Frequenz des vom RFID-Lesegeräts ausgehenden elektromagnetischen Feldes zu arbeiten. Übliche Frequenzen sind dabei 125 kHz, 13,56 MHz (NFC), 868 MHz und 2,45 GHz. Vorzugsweise liegt die Antenne als Rahmenantenne vor. Auf diese Weise kann ein Großteil des Gehäuseinnenraums oder bei Ausführungsformen mit Basis ein Großteil der Basisgrundfläche zur Umwandlung des vom Lesegerät ausgehenden elektromagnetischen Feldes genutzt werden. Dies führt zu einer stabileren kabellosen Verbindung und erhöht deren Reichweite. Da der Benutzer die exakte Position der mobilen Druckmesseinheit unterhalb des Kompressionstextils mitunter nur schwer ausmachen kann, führt eine gute Leistungsfähigkeit der Antenne zu besserem Bedienkomfort.

In Ausnahmefällen kann der RFID-Chip Teil der Leiterplatine sein. In diesem Fall ist es ausreichend eine Antenne an die Leiterplatine anzuschließen, um die beiden Komponenten Leiterplatine und RFID-Transponder in miteinander verbundener Weise zu erhalten.

Darüber hinaus kann die Leiterplatine einen Mikrocontroller umfassen, der operativ mit den beiden Drucksensoren verbunden ist. Gemäß einer bevorzugten Ausführungsform enthalten sowohl der erste als auch der zweite Drucksensor jeweils einen Mikrocontroller, so dass jeder der beiden Drucksensoren ein digitales Signal ausgibt und kein zusätzlicher Mikrocontroller auf der Leiterplatine notwendig ist.

Um den Anpressdruck zu bestimmen, muss der vom ersten Drucksensor generierte Messwert des Außen-Luftdrucks von dem durch den zweiten Drucksensor generierten Messwert des Gehäuse-Innen-Gasdrucks subtrahiert werden. Diese Verrechnung kann sowohl in der mobilen Druckmesseinheit als auch im RFID-Lesegerät stattfinden. Bevorzugt findet die Verrechnung im RFID-Lesegerät statt. Auf diese Weise können Komponenten auf der Leiterplatine eingespart und der Strombedarf vermindert werden.

Die Leiterplatine ist mit dem Gehäuse fest verbunden. Die Verbindung sollte vorzugsweise eine nicht elektrisch leitende Verbindung sein, also einen Isolator darstellen. Da das Gehäuse in den meisten Ausführungsformen selbst ein Isolator ist (abhängig vom verwendeten Material), ist dies jedoch nicht immer zwingend erforderlich. Die Leiterplatine kann mit dem Gehäuse beispielsweise mittels Steckverbindungen, Kunstharz, Klebstoff oder einem Klebeband verbunden werden. Bei dem Klebeband handelt es sich bevorzugt um ein beidseitig klebendes Klebeband. Wenn die Leiterplatine kompakt ausgestaltet ist, - beispielsweise, wenn ihre Längsachse eine Länge von maximal 6 cm hat - ist die Befestigung mit einem doppelseitigen Klebeband die bevorzugte Verbindungsart. Da das Klebeband im inneren des Gehäuses liegt, muss der im Klebeband befindliche Klebstoff nicht zwingend biokompatibel sein. Diese Art der Befestigung erlaubt es die Leiterplatine schnell, kostengünstig und mit geringem Aufwand ins Gehäuse zu integrieren bzw. an der Gehäuseinnenseite zu fixieren. Bevorzugt hat das Klebeband (in der Aufsicht) dieselben Ausmaße wie die Leiterplatine. Dies hat den Vorteil, dass das Klebeband bündig in einem Rahmen positioniert werden kann, welcher ebenfalls die Maße der Leiterplatine hat. Weiterhin bevorzugt hat das Klebeband eine Aussparung bzw. ein Loch, das beim Anbringen des Klebebands in die Basis mit der Öffnung für den ersten Drucksensor überlappt und diese gasdicht umschließt. Auf diese Weise kann der erste Drucksensor durch das Loch im Klebeband hindurchgeführt und in der Öffnung positioniert werden. Ein Austritt von Gas aus dem Gehäuse über die Öffnung wird auf diese Weise verhindert und gleichzeitig kommuniziert der erste Drucksensor mit der atmosphärischen Luft. Besonders bevorzugt erfüllt das Klebeband beide der zuletzt genannten Merkmale (Ausmaße wie Leiterplatine und Loch für ersten Drucksensor).

Als Lesegerät kann jedes aktive RFID-fähige Gerät dienen, das über eine elektrische Energiequelle verfügt oder an eine solche angeschlossen ist und in der Lage ist, ein elektromagnetisches Feld zu erzeugen, welches von der Antenne der mobilen Druckmesseinheit genutzt werden kann, um einen elektrischen Strom zu erzeugen. Daneben muss das Lesegerät in der Lage sein die von der mobilen Druckmesseinheit erzeugten Messwerte, welche die Druckmesseinheit durch Manipulation des elektromagnetischen Feldes kontaktlos sendet, zu empfangen. Optional kann das Lesegerät über eine optische Anzeige verfügen, um die gelesenen Werte auszugeben. Alternativ kann das Lesegerät über einen dauerhaften, beschreibbaren Speicher verfügen, so dass die ausgelesenen Werte an eine andere Vorrichtung übertragen und dort ausgegeben werden können. Bei dem RFID-fähigen Lesegerät kann es sich um ein NFC-fähiges Lesegerät handeln. Beispiele für häufig genutzte NFC-fähige Lesegeräte sind Smartphones. So unterstützen die Betriebssysteme Android (ab Version 4) und iOS (ab Version 13) eine NFC-Kommunikation, sofern das zugehörige Smartphone über die entsprechende Hardwareausstattung verfügt.

Das Lesegerät sollte mit der notwendigen Software ausgestattet sein, um den Auslesevorgang zu starten und die übermittelten Messwerte anzuzeigen. Im Falle eines Smartphones kann eine solche Anwendung als App ausgestaltet sein. So ist es möglich in den Programmiersprachen Kotlin (für Android) oder Swift (iOS) passende Anwendungen zu erstellen.

Bevorzugt beinhaltet das Gehäuse der mobilen Druckmesseinheit neben der flexiblen Kuppel eine Basis. Die Basis liegt der flexiblen Kuppel gegenüber und hat eine der Kuppel zugewandte Innenseite sowie eine der Kuppel abgewandte Außenseite. Die Basis ist mit der Kuppel gasdicht verbunden, so dass zwischen Basis und flexiblem Bereich der gasgefüllte Hohlraum ausgebildet wird. Eine gasdichte Verbindung kann beispielsweise durch Schweißen, durch Kleben, durch Einsatz von Kunstharz, Vulkanisieren oder durch den Einsatz von gasdichtem Klebeband erzeugt werden. Somit beinhaltet die Erfindung eine Form der mobilen Druckmesseinheit, bei der die flexible Kuppel und die Basis gasdicht miteinander verschweißt, verklebt oder vulkanisiert sind. Bevorzugt sind flexible Kuppel und Basis miteinander verschweißt. Besonders bevorzugt sind sie ultraschallverschweißt. Das Verschweißen mittels Ultraschall ist bevorzugt, da hierdurch Verdickungen und scharfe Kanten im Bereich der Schweißnaht vermieden werden und das Hautgefühl beim Späteren anlegen deutlich verbessert wird. Bei Bedarf können sämtliche elektronischen Komponenten (Leiterplatine, Transponder, Drucksensoren) an der Basis befestigt werden.

Beim Vulkanisieren entsteht eine aus Gummi bzw. eine aus einem Elastomer bestehende Verbindung zwischen der Kuppel und der Basis.

Weiterhin können Kuppel und Basis miteinander verklebt werden. Ein Beispiel für geeignete Klebstoffe sind chemisch härtende Klebstoffe. Hierzu gehören u.a. Epoxidharz-Klebstoffe oder Polyurethan-Klebstoffe.

Eine Verbindung von Kuppel und Basis mithilfe geeigneter Kunststoffe ist ebenfalls möglich. Zu den geeigneten Kunststoffen gehören neben den oben erwähnten Elastomeren auch die meisten Thermoplasten wie beispielsweise ABS (Acrylnitril-Butadien-Styrol-Copolymere) oder PLA (Polylactide), wobei im Falle von PLA eine Beimischung mindestens eines amorphen Polymers wie beispielsweise Polyvinylchlorid, Polystyrol, Polycarbonat oder Polymethylmethacrylat empfohlen wird.

Die Basis bietet den Vorteil, dass Komponenten, die im Gehäuseinneren verbaut werden, an der Innenseite der Basis befestigt werden können. Hierzu gehört insbesondere die Leiterplatine. Dies erleichtert den Herstellungsprozess und schützt die verbauten Komponenten.

Bevorzugt ist die Basis der mobilen Druckmesseinheit elastisch. Besonders bevorzugt ist sie gummielastisch. Es wird empfohlen die Basis derart auszugestalten, dass sie einen Shore-A-Wert von 20 bis 80 aufweist. Bevorzugt weist die Basis einen Shore-A-Wert von 25 bis 75, besonders bevorzugt von 30 bis 70 und ganz besonders bevorzugt von 40 bis 60 auf. Im Optimalfall enthält die mobile Druckmesseinheit eine Basis die gummielastisch ist und gleichzeitig einen Shore-A-Wert innerhalb eines der angegebenen Bereiche hat. Eine solche Basis schmiegt sich beim Anpressen während der Kompression an die Haut an und verursacht beim Benutzer keine unangenehmen Empfindungen, wie sie z.B. bei der Verwendung von z.B. scharfkantigen Materialien entstehen. Somit ist eine derartige Basis vorteilhaft für die langfristige Druckmessung während der Kompression und erhöht gleichzeitig die Therapie-Compliance. Gleichzeitig kann sich die Basis den Körperkonturen anpassen.

Beim Einsatz einer gummielastischen Basis wird empfohlen diese planar bzw. im Wesentlichen planar auszugestalten. Auf diese Weise bleibt die Basis am besten an unterschiedliche Körperkonturen anformbar. Sollte jedoch eine nichtelastische Basis gewählt werden, so kann diese für die Anwendung in der Kompressionstherapie am Bein leicht gewölbt bzw. gebogen (bezogen auf die Querschnittsansicht) hergestellt werden. Eine solche Form lässt sich auch ohne elastisch zu sein, passend an das zu behandelnde Bein anlegen. Bevorzugt ist die Basis steifer als die flexible Kuppel. Dies kann erreicht werden durch die Auswahl der Materialien und der Schichtdicke der ausgewählten Materialien. Besonders bevorzugt liegt dabei der Shore-A-Wert der Basis über demjenigen der flexiblen Kuppel. Dies hat den Vorteil, dass sich unter Druckeinfluss zunächst die Kuppel (reversibel) verformt. An der Innenseite der Basis befestigte Komponenten wie z.B. die Leiterplatine bleiben auf diese Weise geringeren Kräften ausgesetzt und somit mechanisch geschützt.

Bevorzugt enthält die Basis Silikon oder ein Polyurethan wie z.B. TPU oder ist mit diesen Materialien beschichtet. Diese Materialien sind hautfreundlich, lassen sich gasdicht ausgestalten, schnell und präzise verarbeiten und sind beständig gegenüber äußeren Einflüssen, wie Schweiß, Wasser, Wundsalben und Desinfektionsmittel. Zudem können sie leicht gereinigt werden. Gemäß einer Ausführungsform ist das Silikon ein Silikonkautschuk oder ein Silikonelastomer. Diese Materialien haben den Vorteil, dass sie Aufgrund eines hohen Reibungskoeffizienten ein Verrutschen der mobilen Druckmesseinheit bzw. ein Verrutschen der Kompressionsbinde auf der Druckmesseinheit verhindern. Bevorzugt handelt es sich um ein Silikonkautschuk oder ein Silikonelastomer, das auf sich selbst einen Reibungskoeffizienten µ > 1 hat. Eine Basis, die TPU enthält oder daraus besteht, bietet darüber hinaus den Vorteil, dass TPU sich besonders gut schweißen lässt und somit auf effiziente und kostengünstige Weise eine gasdichte Verbindung zwischen Basis und Kuppel geschaffen wird.

Bevorzugt haben Basis und flexible Kuppel eine glatte Oberfläche, so dass eine Vermehrung von Bakterien in den Materialien ausgeschlossen wird. Es ist jedoch möglich die Oberfläche mit einer dreidimensionalen Struktur zu versehen, um die Wahrscheinlichkeit eines Verrutschens zu reduzieren. Faserige Strukturen sind technisch möglich, lassen sich jedoch schlecht abwischen, können bei Kontakt mit Feuchtigkeit nass werden, von Mikroorganismen besiedelt werden oder sich elektrostatisch aufladen, was die Funktionsfähigkeit der Elektronik beeinflussen kann, weswegen von der Verwendung von Fasern abgeraten wird. Gemäß einer bevorzugten Ausführungsform der Erfindung enthält die mobile Druckmesseinheit deswegen keine Fasern und /oder Textilien wie beispielsweise gewebte Stoffe oder Vliese. Falls Fasern oder Textilien verwendet werden, wird empfohlen diese an der Außenseite mit Silikon oder Polyurethan zu beschichten.

Bevorzugt hat das Gehäuse eine Öffnung, in welcher der erste Drucksensor befestigt oder eingelassen ist. Diese Öffnung kann sich in der Basis befinden. Dies hat den Vorteil, dass der erste Drucksensor kaum absteht und die Wahrscheinlichkeit, dass dieser durch Kompressionstextilien (z.B. medizinische Binde) beschädigt wird, deutlich reduziert ist. Die Öffnung kann sich in einer an der Außenseite befindlichen Erhebung des Gehäuses bzw. der Basis befinden. Die Erhebung bildet auf diese Weise einen schützenden Rand, um den Sensor. Die Erhebung kann beispielsweise radiär oder kegelförmig sein. Sie kann jedoch auch an die Umrisse des Sensors angepasst sein und somit als Fassung vorliegen. Auch die Höhe der Erhebung kann an die Dimensionen des Sensors angepasst sein. Sie kann beispielsweise eine Höhe von 0,2 bis 3 mm, besser 0,3 bis 2 mm, noch besser 0,4 bis 1 mm aufweisen, gemessen von der Grundfläche des Gehäuses bzw. der Basis aus. Sollte die Grundfläche des Gehäuses bzw. der Basis nicht planar sein, so wird die Höhe ausgehend von demjenigen Bereich der Grundfläche gemessen, welcher an die Erhebung angrenzt. Bevorzugt ist die Öffnung in der Basis rund. Besonders gut geeignete Durchmesser für eine runde Öffnung sind 0,1 - 5 mm sowie 0,3 - 4 mm und 0,5 - 3 mm. Kleinere Öffnungen haben den Vorteil, dass weniger Fläche des zweiten Drucksensors exponiert ist. Zudem verringern kleinere Öffnungen die Wahrscheinlichkeit des Eindringens von Fremdsubstanzen wie Feuchtigkeit (z.B. in Form von Schweiß) in das Gehäuseinnere. Auch können kleinere Öffnungen einfacher gasdicht ausgestaltet werden, was sichere sowie präzise Messungen gewährleistet. Ferner können die Maße der Öffnung derart ausgestaltet sein, dass die Öffnung den ersten Drucksensor oder den Teil des Sensors, der in die Öffnung eingeführt wird, passgenau umschließt.

Wichtig ist, dass der erste Drucksensor derart in der Öffnung der Basis befestigt oder eingelassen ist, dass das Gehäuse weiterhin gasdicht ist und kein Gas aus dem Hohlraum über die Öffnung austreten kann. Der erste Sensor kann durch Ausbildung von chemischen Bindungen gasdicht in der Öffnung befestigt werden. Dabei können unterschiedliche Substanzen eingesetzt werden. Beispielsweise kann die Befestigung des ersten Drucksensors in der Öffnung der Basis durch Epoxidharz vermittelt werden. Epoxidharz ist für Gaspartikel undurchlässig und gewährleistet, dass das Gehäuse auch nach Befestigung des Sensors in der Öffnung weiterhin gasdicht ist. Weiterhin können zum Einsatz kommen verschiedene Formen von Epoxidharzen einschließlich Bisphenol-basierte Epoxid-Harze, aliphatische Epoxidharze, Novolak-Epoxidharze und halogenierte Epoxidharze. Bisphenol-basierte Epoxid-Harze können von dieser Aufzählung ausgenommen sein, da zum einen die Unbedenklichkeit von Bisphenol für den menschlichen Körper bei Kontakt mit der Haut nicht zweifelsfrei bewiesen ist und aus diesem Grund auch die Verarbeitung Bisphenol-basierter Epoxid-Harze strengere Sicherheitsvorkehrungen notwendig machen kann. Ein Vorteil von Epoxidharzen ist, dass sie im Ausgangszustand bereits eine grundlegende Flexibilität aufweisen. Diese kann weiter gesteigert werden, indem dem Epoxidharz ein oder mehrere Weichmacher zugesetzt werden. Beispiele für geeignete Weichmacher sind Ester mehrbasischer Carbonsäuren, mehrwertige Alkohole, Kautschukabbauprodukte, Umsetzungsprodukte von Chlorkohlenwasserstoffen mit Alkalisulfiden. Die Weichmacher können in einem Massenanteil von 1 - 30% im resultierenden Plastisol, also dem mit Weichmacher versetzten Kunstharz, enthalten sein. Weiterhin kann die gasdichte Verbindung ebenfalls durch andere Kunstharze wie z.B. Polyesterharz, Polyurethanharz, Polyharnstoffharz oder Silikonharz, durch Teflon, durch Gummi oder durch Klebstoffe erzeugt werden. Zu den möglichen Klebstoffen gehören - neben den bereits erwähnten Epoxid-Harzen - beispielsweise chemisch härtende Klebstoffe wie Polyurethan-Klebstoffe, Phenol-Formaldehydharz-Klebstoffe, Cyanacrylat-Klebstoffe und Methylmethacrylat Klebstoffe. Polyurethan-Klebstoffe sind die bevorzugten Klebstoffe, da sie nach dem Aushärten eine hohe Flexibilität zeigen und eine hohe Widerstandskraft gegenüber Verbiegungen, wie sie beim Fixieren des Gehäuses der mobilen Druckmesseinheit am menschlichen Körper entstehen können.

Je nach Ausführungsform kann die Öffnung für den ersten Drucksensor zusätzlich mit einer Halterung für den ersten Drucksensor kombiniert werden. Dies ist insbesondere dann der Fall, wenn die Öffnung nicht in der Basis des Gehäuses untergebracht ist. Die Halterung und die Öffnung können ineinander übergehen. Darüber hinaus kann die Öffnung auch in die Halterung integriert sein und einen Teil der Halterung bilden. Die Halterung kann aus einem Kunststoff wie TPE bestehen.

Wenn elektronische Komponenten mittels Epoxidharz an der Innenseite des Gehäuses bzw. der Basis befestigt werden, so ist es möglich diese Befestigung zu stärken, indem die Innenseite ein oder mehrere Einkerbungen aufweist. Diese Einkerbungen befinden sich an der Befestigungsposition und sind um die zu befestigende Komponente herum angeordnet. Das Epoxidharz umschließt oder überlagert die zu befestigende Komponente ganz oder teilweise und füllt die besagten Einkerbungen. Auf diese Weise wird die Kontaktfläche vergrößert und die Befestigung verstärkt. Demgemäß kann die erfindungsgemäße mobile Druckmesseinheit ein bis zwölf Einkerbungen auf der Gehäuseinnenseite, beispielsweise auf der Innenseite der Basis, umfassen. Soll nur eine Einkerbung vorhanden sein, so kann diese ringförmig um die zu befestigende Komponente angeordnet sein. Jede Einkerbung kann eine Tiefe von 0,1 - 8 mm, bevorzugt 0,3 - 5 mm, besonders bevorzugt 0,5 - 3 mm, gemessen vom unmittelbar angrenzenden Rand der Gehäuseinnenseite aufweisen.

Daneben kann der erste Drucksensor mittels eines Klebestreifens, bevorzugt eines beidseitig klebenden Klebestreifens, eines Klebebandes, bevorzugt eines beidseitig klebenden Klebebandes, oder eines Transferbandes in der Öffnung befestigt werden. Beispielsweise kann ein an der Innenseite des Gehäuses bzw. der Basis überstehender Teil des Sensors (über die Öffnung überstehend) mit dem Klebestreifen, dem Klebeband oder dem Transferband befestigt sein. Klebestreifen, Klebeband oder Transferband müssen keine durchgehend geschlossene Fläche haben, sondern können eine Aussparung in den Maßen der Öffnung aufweisen, so dass an der Innenseite des Gehäuses bzw. der Basis eine die Öffnung umrandende Klebefläche geschaffen wird. Daneben kann der Klebestreifen oder das Klebeband den Drucksensor zur Fixierung auch überlagern. Bevorzugt ist bei der fertig montierten mobilen Druckmesseinheit die Öffnung für den ersten Drucksensor von einem der zuletzt genannten Klebemittel umschlossen, so dass sich das Klebemittel lateral zur Öffnung erstreckt und die Leiterplatine (mitsamt erstem Drucksensor) oberhalb der Öffnung liegt. Auf diese Weise wird die Öffnung gasdicht umschlossen und die Gasmenge im Inneren des Gehäuses bleibt auch unter Einwirkung von Außendruck konstant.

Alternativ kann der erste Drucksensor auf die Leiterplatine aufgesteckt und die Leiterplatine mittels des Klebestreifens, des Klebebandes oder des Transferbandes an der Innenseite des Gehäuses, bevorzugt an der Innenseite der Basis, befestigt werden. Wenn Streifen bzw. Band an der Unterseite der Leiterplatine angebracht sind, sollten sie beidseitig klebend ausgestaltet sein. Werden sie an der Oberseite angebracht, so dass sie die Leiterplatine überlagern, können sie auch einseitig klebend ausgestaltet sein. Der auf die Leiterplatine aufgesteckte erste Drucksensor ist in eine Öffnung des Gehäuses, bevorzugt in eine Öffnung der Basis, eingeführt, so dass er mit der atmosphärischen Luft kommuniziert. Diejenige Seite des Sensors, die sich in der Öffnung befindet, liegt dabei in der Regel der Schnittstellenseite, welche mit der Leiterplatine verbunden ist, gegenüber. Die Maße der Öffnung sollten im Wesentlichen derart sein, dass die Öffnung den Sensor passgenau umschließt. Überraschend hat sich gezeigt, dass auf diese Weise der erste Drucksensor gasdicht befestig werden kann. Der Vorteil ist, dass diese Art der gasdichten Befestigung den Arbeitsaufwand verringert und zusätzliches Material (wie z.B. Epoxidharz oder Schrauben) einspart. Darüber hinaus wird durch die Vermeidung von Schrauben die strukturelle Integrität des Gehäuses bewahrt und Sollbruchstellen vermieden. Gemäß einer bevorzugten Variante dieser Befestigung sind mindestens 90%, besonders bevorzugt 95% der Unterseitenfläche der Leiterplatine über einen beidseitig klebenden Klebestreifen bzw. Klebeband an der Basis fixiert.

Besonders bevorzugt sind die beiden Drucksensoren jeweils mit gegenüberliegenden Seiten der Leiterplatine elektrisch verbunden. In dieser Konstellation wird empfohlen die Leiterplatine mit derjenigen Seite, die mit dem ersten Drucksensor verbunden ist, voran, an der Basis zu befestigen.

Zur Befestigung können unter anderem unterschiedliche Klebestreifen verwendet werden (z.B. mit Papierträger oder Gewebeträger) und unterschiedliche Klebebänder (z.B. mit Folienträger aus PP, PET oder PVC), die mit verschiedenen adhäsiven Substanzen (z.B. Acrylkleber) behandelt sind. Wichtig ist, dass die Befestigungsmittel keine elektrische Leitfähigkeit aufweisen. Falls die Leiterplatine über ihre Unterseite befestigt werden soll und diese Unterseite rau oder uneben ist, kann die Verwendung von Transferband gegenüber anderen Befestigungsmitteln vorteilhaft sein, da dieses Unebenheiten besser ausgleichen kann als Klebestreifen oder Klebebänder. Da das Transferband kein Trägermaterial enthält, ist es zudem überaus flexibel.

Wenn die Leiterplatine wie oben ausgeführt mit ihrer Unterseite an der Innenseite des Gehäuses, bevorzugt an der Innenseite der Basis, angebracht ist, kann sie durch einen Rahmen zusätzlich stabilisiert werden. Der Rahmen umfasst eine oder mehrere Erhebungen, die sich an besagter Innenseite befindet bzw. befinden. Der Rahmen kann kontinuierlich oder im Falle mehrerer Erhebungen diskontinuierlich ausgestaltet sein. In diesem Sinne umfasst die Erfindung eine mobile Druckmesseinheit, bei welcher sich die Leiterplatine in einem Rahmen befindet oder von einem solchen umschlossen ist. Vorzugsweise ist der Rahmen aus demselben Material wie das Gehäuse oder wie die Basis. Der Rahmen kann einen Verbund mit dem Gehäuse oder der Basis bilden, beispielsweise indem Gehäuse oder Basis in einem Spritzgussverfahren inklusive Rahmen ausgebildet werden. Alternativ kann der Rahmen auf besagte Innenseite aufgeklebt werden oder als Vertiefung der besagten Innenseite ausgestaltet sein. Wichtig ist, dass der Rahmen an mindestens zwei gegenüberliegenden Positionen Kontakt zu den lateralen gelegenen Flächen der Leiterplatine aufweist, um ein Verrutschen der Leiterplatine bei Krafteinwirkung von außen zu verhindern. Die Höhe des Rahmens kann sich an der Höhe der Leiterplatine orientieren. Bevorzugt hat der Rahmen maximal die Höhe der Leiterplatine. Der Rahmen kann beispielsweise eine Höhe von 0,1 - 5 mm haben, bevorzugt 0,3 - 3 mm und besonders bevorzugt 0,5 - 2 mm. Die Dicke des Rahmens kann beispielsweise 0,2 - 10 mm, bevorzugt 0,4 - 8 mm, besonders bevorzugt 0,5 - 5 mm betragen. Bevorzugt ist der Rahmen ein kontinuierlicher Rahmen, der die gesamte laterale Fläche der Leiterplatine umschließt. Besonders bevorzugt umschließt der Rahmen darüber hinaus auch den ersten Drucksensor, besonders bevorzugt zudem auch die Öffnung, in welche der Sensor eingelassen ist, wobei ein direkter Kontakt zwischen Rahmen und Öffnung nicht notwendig ist, wenn der Sensor auf die Leiterplatine gesteckt ist, da die Platine bereits durch den Rahmen stabilisiert ist.

Ferner können sich auf der Innenseite der Basis vertikale und / oder horizontale Streben befinden. Die Streben stellen statische Verstärkungen der Basis dar. Insofern können sie auch als Verstärkungsstreben bezeichnet werden. Sie können am Boden (Innenseite) der Basis aufsetzen bzw. auf dem Boden der Basis platziert sein. Wenn die Basis sowohl vertikale und horizontale Streben enthält, können diese rechtwinklig zueinander angeordnet sein und bilden dann in der Aufsicht eine gitterartige Waffelstruktur (Netzstruktur). Falls die Basis einen Rahmen für die Leiterplatine hat, können die Streben bis zum Rahmen reichen und optional in den Rahmen übergehen. In diesem Fall bilden die Streben einen Verbund mit dem Rahmen und der Basis. Die Streben bieten den Vorteil, dass die Basis verwindungssteifer wird und dass in Folge von außen einwirkenden Kräften (z.B. beim Anpressdruck während der Kompressionstherapie) zunächst die flexible Kuppel nachgibt. Die elektronischen Komponenten, welche sich im Bereich der Basis befinden, werden gegenüber Verformung und Bruch geschützt. Die Streben können dieselbe Höhe und Dicke haben wie der Rahmen. Die Abstände zwischen den Streben können einen Abstand zueinander von 2 - 10 mm haben, bevorzugt 4 - 8 mm, besonders bevorzugt 5 - 7 mm.

Bevorzugt ist die Leiterplatine flexibel, um sich an Körperkonturen anzupassen und um Beschädigungen bei äußerer Krafteinwirkung (z.B. durch den bei der Kompressionstherapie entstehenden Anpressdruck) zu vermeiden. Besonders bevorzugt sind Gehäuse und Leiterplatine der mobilen Druckmesseinheit beide flexibel. Ganz besonders bevorzugt haben Gehäuse und Leiterplatine der mobilen Druckmesseinheit ein E-Modul von maximal 2 Gpa, besser 0,05 bis 1,8 Gpa, noch besser 0,1 bis 1,3 Gpa und am besten 0,2 bis 1 Gpa. Nach einer weiteren Ausführungsform hat das Gehäuse ein höheres E-Modul als die Leiterplatine. Auf diese Wiese wird der Großteil der von außen einwirkende Kräfte durch das unempfindliche Gehäuse absorbiert und nur ein Bruchteil dieser Kräfte an die empfindliche Leiterplatine weitergegeben.

Bevorzugt ist die Leiterplatine flexibel und hat einen Shore-A-Wert, der kleiner oder gleich dem Shore-A-Wert des Gehäuses oder der Basis ist. Auf diese Weise werden von außen auf die mobile Druckmesseinheit einwirkende Kräfte zum Großteil absorbiert. Beispielsweise kann die Leiterplatine einen Shore-A-Wert von 19 - 79, bevorzugt 24 - 74, besonders bevorzugt 29 - 69 und ganz besonders bevorzugt 39 - 59 haben.

Die Flexibilität und / oder die oben genannten Shore-A-Werte der Leiterplatine lassen sich realisieren durch Verwendung einer Leiterplatine, die Polyimide enthält. Die Polyimide sollten als flexibles Polymer vorliegen. Die Flexibilität kann optimiert werden, indem der überwiegende Teil der Leiterplatine aus Polyimiden besteht. Bevorzugt besteht die Leiterplatine zu mindestens 90% aus Polyimiden oder der elektrisch nichtleitende (elektrisch isolierende) Anteil der Leiterplatine besteht aus Polyimiden. Beispiele für verwendbare Polyimide sind Polysuccinimid, Polybismaleinimid, Polyimidsulfon, Polyetherimide und Polymethacrylimid. Eine Leiterplatine, die Polyimide enthält, ist durch ihre flexiblen Eigenschaften gut geeignet, um sich innerhalb des Gehäuses an Körperkonturen anzupassen und weist bei Einfluss durch äußere Kräfte eine hohe Bruchbeständigkeit auf.

Bevorzugt umfasst die mobile Druckmesseinheit gemäß der vorliegenden Erfindung keine außerhalb des Gehäuses liegenden Kabel. Stattdessen befindet sich in der Regel die gesamte Elektronik im Inneren des Gehäuses. Lediglich der erste Drucksensor benötigt Kontakt zum Außenbereich, da er den Außenluftdruck misst. Bevorzugt befindet sich mindestens 90%, bevorzugt 95%, besonders bevorzugt 99% des Volumens des ersten Drucksensors innerhalb des Gehäuses. Ganz besonders bevorzugt befindet sich der gesamte erste Drucksensor innerhalb des Gehäuses und steht lediglich über eine Öffnung im Gehäuse in Kontakt mit dem Außenluftdruck. Der Vorteil dieser Ausgestaltung liegt in dem besonders guten Schutz der Elektronik. Da Kabel auf der Haut vom Benutzer darüber hinaus als störend empfunden werden können, erhöht sich zudem der Tragekomfort.

Die erfindungsgemäße mobile Druckmesseinheit ermöglicht es auf einen Lagesensor (z.B. Gyrometer, Magnetometer) zu verzichten. Die Druckmessung kommt ohne Lagesensor aus.

Dementsprechend beinhaltet der Erfindung eine mobile Druckmesseinheit die keinen Lagesensor enthält. Gemessen wird der Anpressdruck unabhängig von der Körperposition des Benutzers. Der Vorteil ist ein geringerer Energiebedarf sowie eine kompaktere Bauweise. Um die einzelnen Messwerte untereinander vergleichen zu können, z.B. um festzustellen, ob eine Entstauung des Gewebes unter Kompressionstherapie stattgefunden hat, kann der Benutzer während jeder Messung dieselbe Körperposition einnehmen (z. B. immer im Liegen oder immer im Sitzen).

Bevorzugt enthält die Druckmesseinheit keinen Speicher für elektrische Energie. Unter einem solchen Speicher werden solche Komponenten verstanden, die elektrische Energie chemisch speichern (z.B. Batterien oder Akkumulatoren). Andere Arten von Speicher für elektrische Energie (z.B. Kondensatoren) hingegen können Bestandteil der mobilen Druckmesseinheit sein. Der damit einhergehende Vorteil ist, dass die Druckmesseinheit nicht auf eine interne Stromversorgung angewiesen ist, die sich erschöpfen könnte. Da Kompressionsbinden in der Regel von medizinischen Fachpersonal angelegt werden, ist es für den Benutzer bzw. Patienten unzumutbar die Binde abzunehmen, um die Energieversorgung sicherzustellen. Die mobile Druckmesseinheit gemäß der vorliegenden Erfindung wird insbesondere von außen über das elektromagnetische Feld des Lesegeräts mit Energie versorgt. Die Versorgung des Lesegeräts mit elektrischer Energie kann sichergestellt werden, ohne die zur Kompression verwendeten Textilien entfernen zu müssen. Dementsprechend beinhaltet die Erfindung gemäß dieser bevorzugten Ausführungsform eine mobile Druckmesseinheit, die keinen Speicher für elektrische Energie enthält.

Bevorzugt handelt es sich bei dem RFID-kompatiblen Transponder der mobilen Druckmesseinheit um einen passiven NFC-kompatiblen Transponder. Die dabei verwendete Frequenz liegt bei 13,56 MHz. Vorteil eines solchen NFC-basierten Ansatzes ist, dass die meisten modernen Smartphones über NFC verfügen und als Lesegerät im Rahmen dieser Erfindung fungieren können. Darüber hinaus ist es über NFC nicht nur möglich den Messwert des Anpressdrucks mithilfe des Lesegerätes auszulesen, sondern auch Daten an die mobile Druckmesseinheit zu senden und dort in einem optional vorhandenen Speicher abzulegen. Hierzu kann die erfindungsgemäße mobile Druckmesseinheit über einen beschreibbaren internen Speicher (Read-write memory) verfügen, der z.B. Bestandteil der Leiterplatine oder des Transponders ist. Der Speicher kann z.B. eine Kapazität von mindestens 128 kb haben. In dem Speicher kann beispielsweise festgehalten werden: Anzahl der Messungen, Datum und Uhrzeit der Messung und Druckwert. Der Vorteil ist, dass das medizinische Personal mithilfe der gespeicherten Werte feststellen kann, ob eine Entstauung des Gewebes stattgefunden hat. Im Falle einer Entstauung kann z.B. die Art der Kompressionsbinde getauscht werden oder eine Kompressionsbinde durch einen Kompressionsstrumpf ersetzt werden.

Die erfindungsgemäße mobile Druckmesseinheit kann eine an der Außenseite befindliche Klebeschicht umfassen. Die Klebeschicht kann sich an der Außenseite der flexiblen Kuppel und / oder der Basis befinden und kann durch eine ablösbare Abdeckfolie abgedeckt sein. Die Abdeckfolie kann eine Abzugslasche haben. Die Klebeschicht kann dazu dienen die mobile Druckmesseinheit an der Haut zu fixieren oder so lange zu positionieren, bis die Druckmesseinheit durch das Kompressionstextil überlagert und auf diese Weise fixiert ist. Bevorzugt ist die Klebeschicht als beidseitig klebende Folie ausgestaltet.

Eine Klebeschicht, die sich an der Außenseite der Basis befindet, sollte eine hautkompatible Klebeschicht sein. Eine bevorzugte hautkompatible Klebeschicht ist eine Klebeschicht, die Silikonkleber oder Acrylkleber umfasst oder aus einem dieser Kleber besteht. Eine Klebeschicht, die sich auf der Außenseite von Kuppel oder Basis befindet, kann auch dazu dienen, die mobile Druckmesseinheit an das Kompressionstextil anzukleben. Zum Beispiel kann die mobile Druckmesseinheit zunächst mit der Kuppel voran auf der Haut positioniert bzw. angeklebt werden. Anschließend wird sie mit einer Kompressionsbinde umwickelt, die an einer Klebeschicht der Basis anhaftet. Bevorzugt befindet sich die Klebeschicht an derjenigen Seite der mobilen Druckmesseinheit, an welcher die Öffnung für den ersten Drucksensor liegt, also an derjenigen Seite, die bei Benutzung von der Haut weg zeigt.. Der Vorteil hierbei ist, dass die mobile Druckmesseinheit über eine Adhäsion an das Kompressionstextil fixiert wird, aber die Haut unter der Druckmesseinheit sich weiterhin bewegen kann. Beispielsweise kann die Haut sich bei Bewegungen des Nutzers dehnen oder zusammenziehen, ohne dass die mobile Druckmesseinheit verrutscht. Zusammengefasst kann gesagt werden, dass die mobile Druckmesseinheit, wenn sie besagte Klebeschicht enthält, mittels dieser Klebeschicht an der Haut eines Benutzers und / oder an einer Kompressionsbinde fixiert werden kann.

Bevorzugt kann die flexible Kuppel der mobilen Druckmesseinheit aus einer einzigen Schicht von einheitlicher Dicke bestehen. Geringe produktionsbedingte Abweichungen können vorkommen. Somit kann die flexible Kuppel aus einer einzigen Schicht von im Wesentlichen einheitlicher Dicke bestehen. Die eine einzige Schicht kann ein Polymer wie beispielsweise ein Thermoplast sein. Es kann sich allerdings auch um ein Gemisch aus zwei oder mehr Substanzen handeln. Beispielsweise können Additive vorhanden sein. Der Vorteil der Vermeidung mehrerer Schichten ist, dass eine Delaminierung unter Belastung ausgeschlossen wird. Durch eine einheitliche Dicke werden Materialschwachstellen vermieden.

Vorzugsweise hat die flexible Kuppel - insbesondere, wenn sie aus einer einzigen Schicht von im Wesentlichen einheitlicher Dicke besteht - eine Schichtdicke von 0,2 - 3 mm, besonders bevorzugt 0,3 - 2 mm und ganz besonders bevorzugt 0,4 - 1 mm. Durch Verwendung einer dünnen Schicht werden genauere Messungen erzielt, da dünne Schichten von außen einwirkende Druckkräfte weniger durch elastische Materialverformung absorbieren und stattdessen besser an den gasgefüllten Hohlraum weitergeben, wo die Druckänderung vom zweiten Drucksensor erfasst werden kann. Darüber hinaus kann die Verwendung von dicken Schichten bei der flexiblen Kuppel zu einem unerwünscht erhöhten Ausgangsdruck im inneren des Gehäuses führen, was durch die Gewichtskraft der flexiblen Kuppel bedingt ist.

Vorzugsweise enthält die flexible Kuppel ein Polyurethan oder besteht aus diesem. Bei dem Polyurethan kann es sich um TPU handeln. Wenn die mobile Druckmesseinheit über eine Basis verfügt, so können sowohl Basis als auch flexible Kuppel aus TPU bestehen. Der Vorteil hierbei ist, dass sich Basis und flexible Kuppel durch Schweißen dauerhaft gasdicht miteinander verbinden lassen und dass sich dieser Schweißschritt auf einfache Weise automatisieren lässt. Es wird empfohlen das Schweißen als Ultraschallschweißen durchzuführen.

Vorzugsweise weist die mobile Druckmesseinheit mindestens eine Symmetrieebene auf. Beispielsweise kann sie in der Aufsicht trapezförmig ausgestaltet sein. Es können auch mindestens zwei Symmetrieebenen vorhanden sein. Beispielsweise kann die mobile Druckmesseinheit in der Aufsicht rechteckig ausgestaltet sein. Es können auch mindestens drei Symmetrieebenen vorhanden sein. Beispielsweise kann die mobile Druckmesseinheit in der Aufsicht quadratisch ausgestaltet sein. Bei den hierbei genannten geometrischen Formen können die Ecken abgerundet sein, da Kanten zu einem unangenehmen Hautgefühl führen. Bevorzugt ist die mobile Druckmesseinheit in der Aufsicht elliptisch oder kreisförmig (radiär) ausgestaltet. Auf diese Weise werden Ecken von vorneherein vermieden und durch den Anpressdruck wird die flexible Kuppel gleichmäßig in alle Richtungen verdrängt, was möglichen Materialschwachstellen vorbeugt.

Vorzugsweise umfasst der Transponder eine Rahmenantenne bzw. eine RFID- oder NFC-Rahmenantenne, die mindestens 15% der Grundfläche der mobilen Druckmesseinheit umschließt. Wenn die Druckmesseinheit über eine Basis verfügt, wird die Grundfläche durch die Unterseite der Basis bestimmt. Größe Rahmenantennen generieren mehr Strom bzw. gewährleisten eine größere Reichweite. In diesem Sinn kann die Rahmenantenne auch mindestens 20%, besser mindestens 30%, noch besser mindestens 40% und am besten mindestens 60% der Grundfläche der mobilen Druckmesseinheit umschließen. Hiermit ist diejenige Fläche gemeint, welche von den Außenmaßen der Rahmenantenne maximal abgedeckt werden kann, also beim flachen Aufliegen (nicht hochkant) des Rahmenanteils der Antenne ohne eventuell vorhandene Anschlüsse etc. Bei mobilen Druckmesseinheiten mit Basis kann sich das eben Beschriebene auch auf die Grundfläche der Basis beziehen. Daneben können vorteilhafte Größen der Rahmenantenne auch in absoluten Werten angegeben werden. In diesem Sinne kann die Rahmenantenne eine Fläche von mindestens 200 mm² abdecken. Eine kreisförmige Rahmenantenne kann einen Durchmesser von mindestens 16 mm haben. Eine quadratische Rahmenantenne kann eine Kantenlänge von mindestens 14 mm haben. Rahmenantennen, die die aufgeführten Dimensionen haben, können sowohl RFID- als auch NFC-fähig sein.

Die mobile Druckmesseinheit ist eine Vorrichtung zur Messung des Anpressdrucks. Andere Messarten können explizit ausgenommen sein. Beispielsweise die Messung des Blutdrucks oder die Messung des Sauerstoffgehalts im Blut. Somit kann die mobile Druckmesseinheit zur Messung des Anpressdrucks keinen Sensor enthalten, der geeignet ist, den Blutdruck oder den Puls oder den Sauerstoffgehalt im Blut zu messen. In den besagten Fällen ist die mobile Druckmesseinheit kein Blutdruckmessgerät bzw. kein Pulsmessgerät oder kein Pulsoxymeter und enthält eine solche Messeinheit auch nicht.

Bevorzugt ist die mobile Druckmesseinheit spritzwassergeschützt. Wenn das Gehäuse eine Öffnung hat, so kann diese Öffnung durch eine gasdurchlässige, aber wasserundurchlässige Membran abgedichtet sein. Dies ist jedoch nicht notwendig, um die Druckmesseinheit spritzwassergeschützt auszugestalten. Stattdessen kann auch eine kleine Öffnung verwendet werden. Eine kleine Öffnung kann eine Fläche von bis zu 10 mm², besser bis zu 8 mm², noch besser bis zu 5 mm² und am besten bis zu 3 mm² haben. Wenn die Öffnung kreisförmig ist, so kann sie einen Durchmesser von bis zu 4 mm haben, besser bis zu 3 mm, noch besser bis zu 2 mm und am besten 1 mm. Bevorzugt ist die mobile Druckmesseinheit nach mindestens IP5X geschützt gegen Staub und Berührung. Daneben kann die mobile Druckmesseinheit geschützt sein gegen Wasser nach mindestens IPX1, besser mindestens nach IPX2, noch besser mindestens nach IPX3 und am besten mindestens nach IPX4.

Die mobile Druckmesseinheit hat darüber hinaus den Vorteil, dass sie desinfizierbar ist mithilfe von Desinfektionsmittel. Dementsprechend ist die mobile Druckmesseinheit desinfizierbar. Beispielswiese mithilfe handelsüblicher Desinfektionsmittel auf Alkoholbasis oder Desinfektionsmittel für die Flächendesinfektion, welche Glutaral, Benzyl-c12-18 alkyldimethylammoniumchloride und / oder Didecyldimethylammoniumchlorid enthalten. Der Vorteil ist, dass ein und dieselbe mobile Druckmesseinheit bei unterschiedlichen Benutzern bzw. Patienten verwendet werden kann, ohne dass es zu einer unbeabsichtigten Verschleppung von Keimen (beispielsweise aus offenen Wunden) kommt. Wenn die mobile Druckmesseinheit desinfiziert werden soll, wird empfohlen eine Druckmesseinheit ohne Klebeschicht zu verwenden.

Bevorzugt befindet sich im gasgefüllten Hohlraum Luft. Es kann jedoch auch ein anderes Gas oder Gasgemisch wie eines oder mehrere Edelgase oder Stickstoff enthalten sein. Bevorzugt hat das Gas im Hohlraum einen geringen Wasserdampfanteil. Der Wasserdampfanteil kann dabei weniger als 6 g Wasser pro m³ Gas bei 20°C sein. Besser weniger als 5 g, noch besser weniger als 4 g und am besten weniger als 3 g. Besonders bevorzugt ist das Gas (z.B. Luft) frei oder im Wesentlichen frei von Wasserdampf. Je trockener das Gas im Hohlraum der Druckmesseinheit, desto geringer ist die Wahrscheinlichkeit, dass es bei Temperaturschwankungen zu einer Kondensation im Inneren des Gehäuses kommt. Dies stellt einen zusätzlichen Schutz für die elektronischen Komponenten dar.

Bevorzugt hat die mobile Druckmesseinheit (ohne optionale Abdeckfolien) oder das Gehäuse eine Höhe von 2 - 8 mm im Ausgangszustand. Ausgangszustand bedeutet hier, dass kein Anpressdruck ausgeübt wird. Besonders bevorzugt hat die mobile Druckmesseinheit eine Höhe von 2,5 - 4 mm im Ausgangszustand. Dies gilt insbesondere, wenn das Gehäuse über eine Kuppel und eine Basis verfügt oder aus diesen besteht. Flache (also niedrige) Ausführungen haben den Vorteil, dass sie unterhalb eines Kompressionstextils nicht auftragen und vom Benutzer nicht als störend wahrgenommen werden.

Bevorzugt sind sowohl der erste und / oder der zweite Drucksensor der mobilen Druckmesseinheit digitale Drucksensoren. Der Vorteil hierbei ist, dass die Leiterplatine nicht mit einem zusätzlichen Analog-Digital-Umwandler verbunden sein muss.

Die Erfindung beinhaltet darüber hinaus ein Set zur Kompressionstherapie umfassend eine medizinische Binde und die beschriebene mobile Messeinheit. Das Set kann weiterhin eine Umverpackung beinhalten, in welcher sich Binde und Messeinheit befinden. Die medizinische Binde ist eine Binde, die zur Kompressionstherapie eingesetzt werden kann und auch als Kompressionsbinde bezeichnet werden kann. Hierbei kann sich um eine kohäsive Binde handeln. Eine solche kohäsive Binde kann einseitig oder beidseitig kohäsiv sein. Ferner kann die Binde ein oder mehrere Materialien aus der folgenden Gruppe enthalten: Polyester, Polypropylen, Viskose, Baumwolle, Polyamid und Elastan. Bevorzugt enthält die Binde Polypropylen und Elastan oder besteht daraus. Das Set kann darüber hinaus auch eine Polsterbinde enthalten, die genutzt wird, um Hautunebenheiten vor der Kompressionstherapie auszugleichen. Die Polsterbinde kann Polyester und Elastan enthalten und kann ebenfalls kohäsiv sein. Die medizinische Binde und die optionale Polsterbinde sind atmungsaktiv, so dass unterhalb der Binden während der Kompressionstherapie der Außen-Luftdruck gemessen werden kann.

Die mit der mobilen Druckmesseinheit verwendbaren medizinischen Binden können beispielsweise einen KADI (Knöchel-Arm-Druck-Index) von 0,6 bis 1,3 haben. Optional können auf der Oberfläche der medizinischen Binden visuelle Anlegeindikatoren vorhanden sein. Die Anlageindikatoren können beispielsweise als Sechsecke ausgestaltet sein. Die Anlegeindikatoren geben bereits während des Anlegens eine visuelle Rückmeldung darüber, ob die Binde korrekt gedehnt wird. Beispielsweise kann sich bei korrekter Dehnung ein ungleichmäßiges Sechseck in ein Sechseck mit gleich langen Seiten verformen. Der genaue Anpressdruck kann im Anschluss mithilfe der mobilen Druckmesseinheit erfasst werden. Derartige Binden mit Anlageindikatoren. können auch in dem beschriebenen Set enthalten sein.

Das Set kann darüber hinaus einen QR-Code enthalten. Der QR-Code kann auf der mobilen Druckmesseinheit, der medizinischen Binde oder der Umverpackung aufgedruckt oder aufgeklebt sein. Ferner kann der QR-Code auf einem in der Umverpackung weiterhin enthaltenen Druckerzeugnis (z.B. einer Bedienungsanleitung) abgebildet sein. Der QR-Code kann eine URL codieren. Bei der URL kann es sich um einen Download-Link für eine Messanwendung bzw. um eine Installationsdatei einer solchen Messanwendung handeln. Alternativ kann über die URL eine Gebrauchsanleitung im Text- oder Videoformat abgerufen werden. Ferner kann der QR-Code eine Zahlenabfolge von z.B. fünf bis zehn Zahlen kodieren, bei der es sich um eine Aktivierungsnummer handeln kann. Die Aktivierungsnummer kann hierbei unikal ausgestaltet sein, so dass jeder mobile Druckmesseinheit eine eigene Aktivierungsnummer über einen QR-Code zugewiesen wird.

Von der Erfindung eingeschlossen ist auch ein Verfahren zur Herstellung der beschriebenen mobilen Druckmesseinheit, bei welchem die hergestellte Druckmesseinheit über eine Basis verfügt und welches die folgenden Schritte umfasst:
a) Bereitstellen der flexiblen Kuppel, der Basis, des ersten und des zweiten, Drucksensors, des RFID-Transponders und der Leiterplatine,
b) Befestigung der Leiterplatine an der Basis,
c) Anschließen der Drucksensoren und des RFID-Transponders an die Leiterplatine, wobei der erste Drucksensor eine Verbindung zur Außenseite der Basis hat
d) Verschweißen der Kuppel mit der Basis, so dass zwischen der Kuppel und der Basis ein gasdichter gasgefüllter Hohlraum ausgebildet wird und so dass Kuppel und Basis den zweiten Drucksensor, den Transponder und die Leiterplatine umschließen.

Die Basis kann im Randbereich ein oder mehrere Aussparungen haben. Die Aussparungen können beispielsweise eine Fläche von 1 × 2 mm haben. Gleichzeitig kann die flexible Kuppel dieselbe Anzahl an Erhebungen haben, welche derart dimensioniert sind, dass sie in die Aussparungen passen. Dies Erleichtert die Herstellung, da die flexible Kuppel und die Basis, während sie aneinander befestigt werden, bereits vorfixiert sind. Nach der Herstellung ergibt sich zudem der Vorteil, dass die Verbindung zwischen Basis und Kuppel belastbarer ist, da sie über eine größere Kontaktfläche zueinander verfügen. Demgemäß ist Teil der Erfindung eine mobile Druckmesseinheit, bei der die Basis eine Anzahl von Aussparungen hat und die Kuppel dieselbe Anzahl von Vorsprüngen (Stiften) und wobei die Vorsprünge passgenau in die Aussparungen eingelassen sind.

Ein Gehäuse der mobilen Druckmesseinheit, das den hier beschriebenen Aufbau hat, ist gasdicht bis zu einem von Außen einwirkenden Druck von mindestens 80 mmHg, besser mindestens 150 mmHg, noch bester 200 mmHg und am besten mehr als 200 mmHg. Somit ist ein Austritt von Gas aus dem Gehäuse während z.B. einer Kompressionstherapie ausgeschlossen.

Weiterhin wird hier ein Verfahren zur Messung des Anpressdrucks während der Kompressionstherapie beschrieben, welches die folgenden Schritte umfasst:
a) Anlegen der mobilen Druckmesseinheit auf die Haut des Patienten in dem zu behandelnden Bereich
b) Überlagern der mobilen Druckmesseinheit mit einer oder mehreren Lagen eines zur Kompression geeigneten Textils, wobei das Textil eine Binde sein kann und in diesem Fall zum Überlagern der zu behandelnden Körperbereich umwickelt wird und das Ende der Binde optional fixiert wird
c) Bereitstellen eines elektromagnetischen Feldes durch ein in der Nähe zur mobilen Druckmesseinheit befindliches Lesegerät zur Stromversorgung der mobilen Druckmesseinheit
d) Messen des Anpressdrucks durch die mobile Druckmesseinheit
e) Auslesen des von der mobilen Druckmesseinheit gemessenen Anpressdrucks mit dem Lesegerät
f) Vergleich des gemessenen Anpressdrucks mit dem gewünschten Anpressdruck
g) Optionale Korrektur des Anpressdrucks durch Austausch, Straffung oder Lockerung des Textils
h) Optionales Wiederholen der Schritte f) und g) bis der gemessene Anpressdruck mit dem gewünschten Anpressdruck übereinstimmt

Das Textil kann in diesem Verfahren beispielsweise ein Kompressionsstrumpf oder eine Kompressionsbinde sein. Der gewünschte Anpressdruck kann auch ein Druckbereich sein. Das Fixieren des Endes der Binde kann beispielsweise mit Klammern oder Klebestreifen erfolgen, wobei Klebestreifen aufgrund des geringeren Verletzungsrisikos der Vorzug zu geben ist. Kohäsive Binden können an sich selbst fixiert werden.

Des Weiteren umfasst die Erfindung die Verwendung der mobilen Druckmesseinheit zum Einstellen des Anpressdrucks während einer Kompressionstherapie oder unter einer Kompressionsbekleidung. Das korrekte Einstellen des Anpressdrucks unter Kompressionsbekleidung kann die Muskelregeneration nach sportlicher Betätigung beschleunigen. Für eine beschleunigte Muskelregeneration sollte ein Kompressionstextilstück gewählt werden, dass einen Druck von von 10 - 32 mmHg, bevorzugt 15 bis 28 mmHg auf die Extremität bzw. auf das Gewebe (z.B. Rumpfmuskulatur) erzeugt. In diesem Sinne umfasst die Erfindung die Verwendung der mobilen Druckmesseinheit zur Beschleunidung der Muskelregeneration bzw. die Verwendung zum Einstellen eines Drucks auf das Muskelgewebe, dass die Regeneration des Muskelgewebes beschleunigt. Ferner ist auch eine Verwendung der mobilen Druckmesseinheit zur Auswahl eines Kompressionstextilstücks, welches den oben genannten Druck auf das Gewebe ausübt, zur Beschleunigung der Muskelregeneration möglich. Vorteilhaft hierbei ist, dass die mobile Druckmesseinheit nach Auswahl des geeigneten Kompressionstextils entfernt werden kann und das Kompressionstextil (z.B. über Nacht) ohne die Druckmesseinheit getragen werden kann.

Schließlich umfasst die Erfindung einen Schuh, bevorzugt einen Sportschuh, dessen Sohle mindestens eine mobile Druckmesseinheit umfasst. Bevorzugt umfasst die Sohle mindestens zwei mobile Druckmesseinheiten. Auf diese Weise kann die Druckverteilung auf die Sohle beim Auftreten gemessen werden. Falls die Messwerte bereits während des Gehens oder Laufens ausgelesen werden sollen, empfiehlt es es sich auf eine andere RFID-Frequenz als NFC auszuweichen, da der NFC-Standard prinzipiell darauf ausgelegt ist nur über kurze Distanzen (etwa 10 cm) zu funktionieren. Alternativ können die Messwerte auch zwischengespeichert und zu einem späteren Zeitpunkt ausgelesen werden.

Nachfolgend werden mehrere konkrete Ausgestaltungen der Erfindung anhand von Beispielen dargestellt. Diese Beispiele zeigen, wie die Erfindung in der Praxis umgesetzt werden kann. Mithilfe der in diesem Dokument dargelegten Informationen können die Beispiele dem jeweiligen Zweck angepasst werden oder es können Ausführungsformen generiert werden, welche von den Beispielen unabhängig sind.

### Beispiele

### Beispiel 1: Montage und Aufbau einer runden mobilen Druckmesseinheit

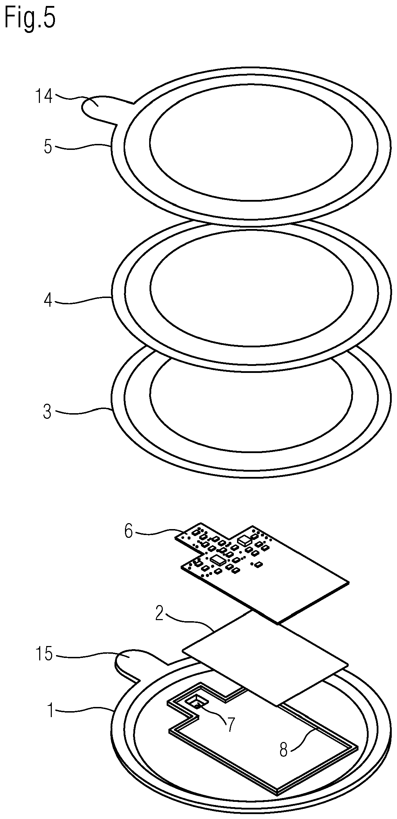

Es wurde eine mobile Druckmesseinheit zusammengesetzt, bei welcher die nachfolgend beschriebenen Komponenten zum Einsatz kamen:
Als Basis 1 diente ein Körper aus Desmopan^{®} DP 6065A Polyurethan des Herstellers Covestro. Es handelte sich um ein PU mit einer Dichte von 1,08 g/cm³ nach ISO 1183 und einem Shore-A-Wert von 66 nach ISO 7619-1. Die Basis 1 war radiär mit einem Durchmesser von 55 mm, einer Materialstärke (Dicke) von etwa 1 mm und hatte eine maximale Höhe von 2 mm. Der Randbereich der Basis 1 war um 1 mm angehoben, parallel zur Grundfläche ausgerichtet (vergleiche Fig. 5) und hatte eine Breite von 3,1 mm, so dass die Basis tellerförmig ausgestaltet war.

Die Basis verfügte über eine durchgehende Öffnung 7 für den ersten Drucksensor, die von der Außen- bis zur Innenseite reichte. Auf der Außenseite war die Öffnung von einer Fassung umgeben, die eine Höhe von 1 mm hatte.

Darüber hinaus hatte die Basis eine Grifflasche 15, an der die Druckmesseinheit nach beendetem Einsatz von der Haut abgezogen werden konnte. Die Lasche 15 hatte eine Länge von 10 mm und die maximale Breite betrug 8 mm. Die Lasche 15 war derart ausgerichtet, dass sie sich 10 mm vom Rand der Basis erstreckte und ihr abstehendes Ende war in der Aufsicht gebogen.

Die Basis 1 verfügte auf ihrer Innenseite über einen durchgehenden Rahmen 8 zur Aufnahme der Leiterplatine 6. Die oben erwähnte Öffnung 7 für den ersten Drucksensor lag ebenfalls innerhalb dieses Rahmens 8. Die vom Rahmen 8 umschlossene Fläche setzte sich zusammen aus einem 26,45 mm x 22,4 mm großen Rechteck und einem 10,75 mm x 9,3 mm großen Quadrat, wobei in der quadratischen Fläche die Drucksensoröffnung 7 lag.

Als Leiterplatine 6 diente ein flexibles PCBA (Printed Circuit Board Assembly) aus beidseitig kupferbeschichtetem Polyimid mit eingebetteter NFC-Antenne 20, auf welches der erste Drucksensor 12 und der zweite Drucksensor 18 aufgesteckt wurden, wobei die beiden Sensoren 12, 18 auf gegenüberliegenden Seiten des PCBA angeordnet wurden. Bei den Sensoren 12, 18 handelte es baugleiche digitale, barometrische Drucksensoren mit Abmessungen von jeweils etwa 2 x 2 x 0,75 mm. Beide Sensoren 12, 18 verfügten über ein piezoresistives Drucksensorelement und eine ASIC (anwendungsspezifische integrierte Schaltung), wobei die ASIC gleichzeitig als analog-digital Wandler fungierte. Das derart bestückte PCBA, wurde mithilfe eines doppelseitig klebenden Klebebands 2 im Rahmen montiert. Das Klebeband 2 hatte die Innenmaße des Rahmens 8, so dass es passgenau in selbigen eingeklebt wurde, wobei ein Loch im Klebeband 2 die Durchführung des ersten Drucksensor 12 von der Leiterplatine in die Öffnung 7 erlaubte. Hierzu wurde die Leiterplatine 6 mit dem ersten Drucksensor 12 voran montiert und der besagte Drucksensor dabei in die hierzu vorgesehene Öffnung 7 platziert. Die Öffnung 7 war anschließend vom Klebeband 2 gasdicht umschlossen. Die Höhe des Klebebands betrug etwa 0,1 bis 0,2 mm.

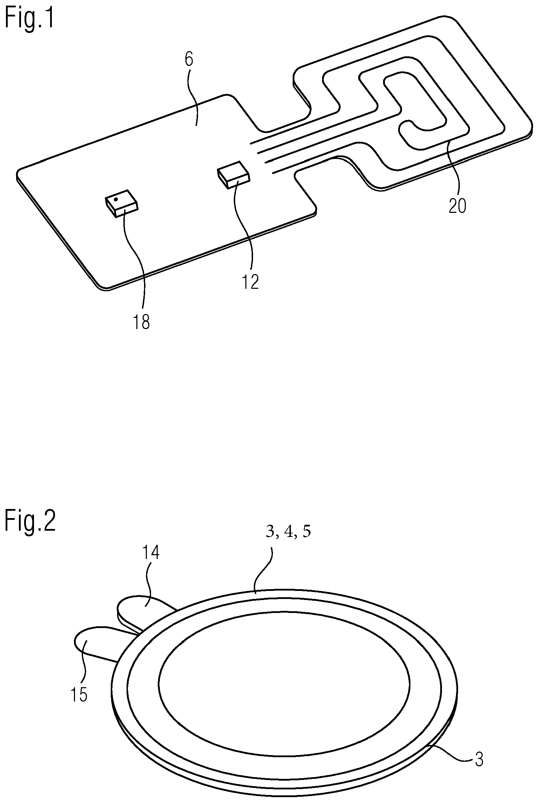

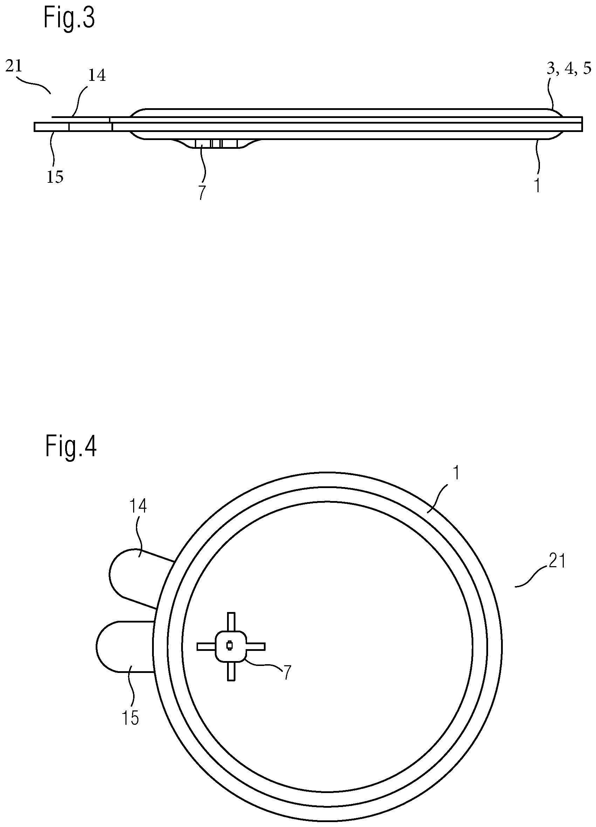

Anschließend wurde eine Kuppel 3 auf die Basis 1 aufgesetzt, so dass Kuppel 3 und Basis 1 sich im oben erwähnten Randbereich der Basis 1 gegenseitig berührten. Dabei wurde die Kuppel 3 mit der konvexen Seite nach außen platziert. Die Kuppel 3 war aus dem gleichen PU-Material gefertigt wie die Basis 1, war ebenfalls radiär und hatte denselben Durchmesser, jedoch keine Grifflasche. Die Kuppel 3 hatte eine Schichtdicke von 1 mm und eine Gesamthöhe von 2 mm. Die Kuppel 3 war an ihrer Außenseite mit einer beidseitig klebenden Folie 4 überlagert. Als Klebemittel kam Acrylkleber zur Anwendung. Die Klebefolie 4 war durch eine Abdeckfolie 5 aus Polypropylen geschützt. Die Abdeckfolie 5 hatte eine überstehende Abzugslasche 14. Kuppel 3 und Basis 1 wurden mittels Ultraschall gasdicht miteinander verschweißt. Eine Prüfung ergab, dass bei einem auf das Gehäuse von Außen applizierten Druck von 200 mmHg kein Gas austrat und das Gehäuse nach Ende der Prüfung wieder seine ursprüngliche Form annahm. Die resultierende mobile Druckmesseinheit hatte den Aufbau wie schematisch in Fig. 5 dargestellt. Eine Detailansicht der Kuppel 3 kann Fig. 2 entnommen werden, während Fig. 1 die Leiterplatine 6 im Detail wiedergibt. Die fertig montierte Druckmesseinheit ist in Fig. 3 in der Seitenansicht abgebildet.

### Beispiel 2: Messung eines Kompressionsdrucks

Die mobile Druckmesseinheit gemäß Beispiel 1 wurde einer Testperson im Wadenbereich an der Position B1 angelegt. Die Position B1 ist ein häufig in der Kompressionstherapie zur Druckmessung genutzter Bereich und ihre Lage ist den in der Kompressionstherapie bewanderten Personen geläufig.

Zum Anlegen wurde die Abdeckfolie 5 von der Klebeschicht 2 der Kuppel abgezogen und die mobile Druckmesseinheit mit der Klebeschicht 2 voran auf die saubere Haut aufgelegt. Über die Wade (mitsamt der daran befestigten Druckmesseinheit) und dem Fuß (bis zu den Zehengrundgelenken) wurde ein Kompressionsverband angelegt. Dazu wurde der Bereich mit zwei Kompressionsbinden mittels gegenläufiger Wickeltechnik umwickelt. Der Fuß befand sich in rechtwinkliger Stellung zum Unterschenkel. Bei der Kompressionsbinde handelte es sich um das Modell "Pütter-Verband^{®}" der Herstellers Paul Hartmann AG - einer Kurzzugbinde aus 100% Baumwolle und einer Dehnbarkeit von ca. 90 %. Jede Binde war 10 cm breit und hatte in voller Dehnung eine Länge von ca. 5 m. Zum Schluss wurde das lose Ende der zuletzt angelegten Kompressionsbinde mittels eines handelsüblichen Pflasterstreifens fixiert.

Mittels Auslesens eines dafür zuvor erstellten QR-Codes mit der Kamera eines Smartphones (Betriebssystem Android Version 13) wurde der Download einer in Kotlin geschriebenen Mess-App auf das Smartphone initiiert. Nach Abschluss der Installation wurde die App gestartet, ein Benutzerkonto angelegt und aktiviert. Das Soll-Druck-Intervall für die Kompressionstherapie wurde in der App vorgegeben. Die Testperson wurde in eine sitzende Position gebracht. Das Smartphone fungierte als Lesegerät und wurde bis auf eine Entfernung von etwa 5 cm an die Position B1 herangeführt, um die mobile Druckmesseinheit mit Strom zu versorgen und die Messung einzuleiten. Der ermittelte Messwert wurde innerhalb weniger Sekunden mittels Smartphone ausgelesen (ebenfalls über NFC, verschlüsselte Übertragung) und auf dem Display des Smartphones in der Einheit mmHg ausgegeben. Gleichzeitig wurde der Messwert von der Anwendung mit dem Soll-Wert-Intervall verglichen und als korrekt ausgegeben. Der Messwert wurde unter TLS-Verschlüsselung an die Cloud-Datenbank MongoDB Atlas geschickt und dort anonymisiert für späteren Abruf gespeichert. Die Messung wurde nach zwei Stunden wiederholt. Dabei befand sich die Testperson in der gleichen Sitzposition wie bei der ersten Messung, um eine Vergleichbarkeit der Messwerte sicherzustellen.

### Beispiel 3: Verstärkte runde mobile Druckmesseinheit

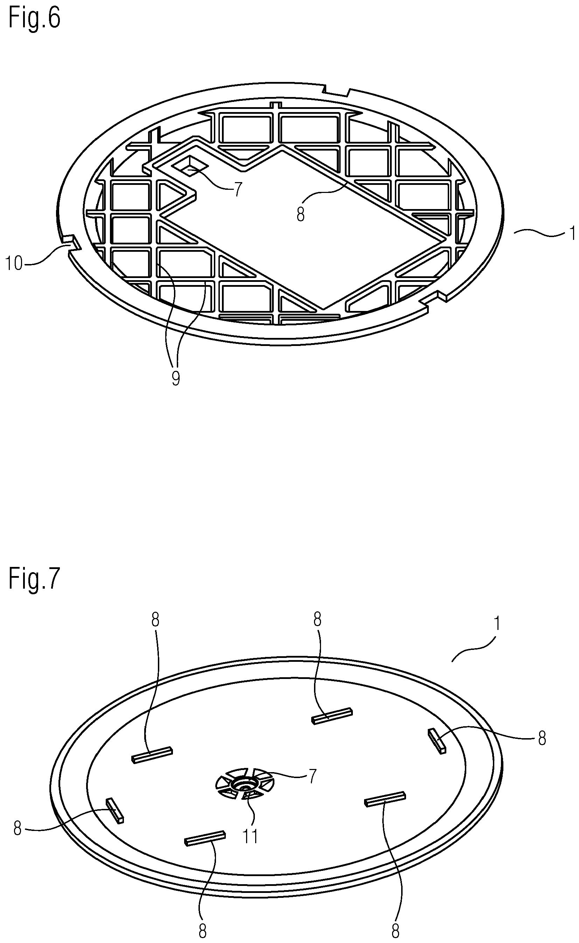

Bereitgestellt wurde eine Basis 1 mit gleichen Abmessungen wie in Beispiel 1 allerdings ohne Grifflasche 15. Der Rahmen 8 auf der Innenseite war zusätzlich von längs- und querverlaufenden Verstärkungsstreben 9 umgeben, die in der Aufsicht eine Waffelstruktur 17 ergaben. Die Verstärkungsstreben 9 waren 1 mm hoch und 1 mm breit, rechtwinklig zueinander und diagonal zum Rahmen 8 angeordnet. Die Innenräume der Rechtecke der Waffelstruktur waren bis zu 7 x 5 mm groß. Der Rand der Basis war zudem mit drei gleichmäßig verteilten Aussparungen 10 mit einer Größe von jeweils 1 × 2 mm versehen. Eine Ansicht dieser Basis kann Fig. 6 entnommen werden.

Die zugehörige Kuppel 3 besaß an der Unterseite ihres Randbereichs drei Vorsprünge in Form von Stiften 22, welche so dimensioniert waren, dass sie sich in die Aussparungen 10 der Basis 1 einführen ließen und mit diesen bündig abschlossen. Die Stifte 22 hatten eine Höhe von jeweils ca. 1 mm. Die übrigen Komponenten und der Schritt des Verschweißens von Basis 1 und Kuppel 3 waren gleich zu Beispiel 1, allerdings wurde auf Klebeschicht 4 und Abdeckfolie 5 auf der Kuppel 3 verzichtet.

### Beispiel 4: Feuchtigkeitsbeständige runde mobile Druckmesseinheit

Bereitgestellt wurde eine Basis 1 mit gleichen Abmessungen wie in Beispiel 1, allerdings ohne Grifflasche 15. Der Rahmen 8 war diskontinuierlich ausgestaltet und bestand aus sechs voneinander getrennten Rahmenelementen. Die Öffnung für den ersten Drucksensor 7 war auf der Innenseite der Basis 1 eingefasst von sechs keilförmigen Einkerbungen 11 für das Einfüllen von Epoxidharz. Eine Ansicht dieser Basis kann Fig. 7 entnommen werden.

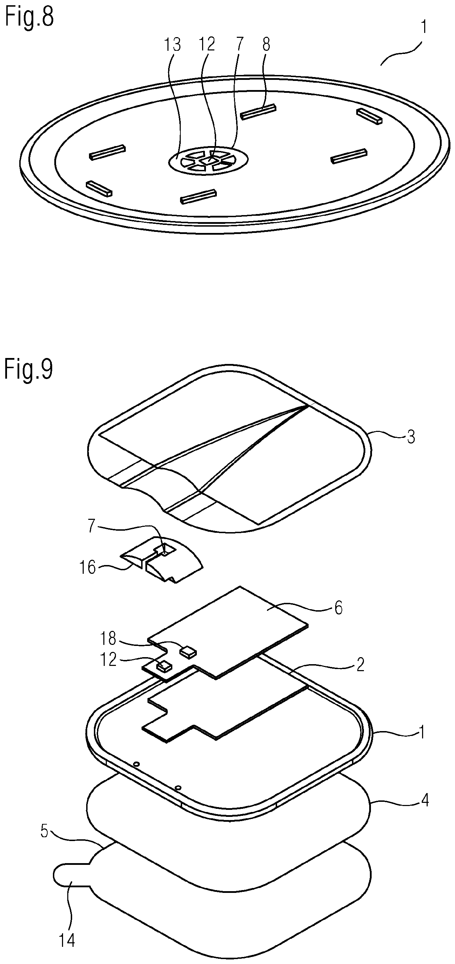

Der erste Drucksensor 12 wurde in die dafür vorgesehene Öffnung 7 eingesetzt. Anschließend wurde in dem Bereich um den Sensor 12 Epoxidharz eingefüllt. Die Füllhöhe war derart gewählt, dass das Harz sich sowohl in als auch oberhalb der Einkerbungen 11 ansammelte und die Einkerbungen 11 durch das Harz miteinander verbunden wurden. Dabei wurde darauf geachtet, dass das Harz nicht den oberen Rand des Sensors 12 oder dessen elektrische Kontakte erreichte. Das Ergebnis ist in Fig. 8 schematisch dargestellt.

Nach dem Aushärten des Epoxidharzes wurde eine Leiterplatine 6 mit eingebetteter NFC-Antenne 20 auf den im Harz verankerten ersten Drucksensor 12 aufgesteckt. Eine Klebeverbindung für die Fixierung der Leiterplatine war nicht notwendig. Die übrigen Komponenten wurden wie in Beispiel 1 dargelegt verbaut. Nach dem Verschweißen von Basis 1 und Kuppel 3 war die mobile Druckmesseinheit einsatzbereit.

### Beispiel 5: Mobile Druckmesseinheit in Form eines abgerundeten Quadrats

Bereitgestellt wurde eine quadratische Basis 1 aus TPE, die an ihrer Außenseite von einer beidseitig klebenden Folie 4 aus Polypropylen überlagert war. Als Kleber wurde Silikonkleber verwendet. Die Klebefolie war an ihrer Außenseite mit einer Abdeckfolie 5 aus Polypropylen inklusive Abzugslasche 14 überlagert. Die Basis verfügte über einen erhabenen Rand, der einen durchgehenden planaren Randbereich bildete.

Der erste Drucksensor 12 und der zweite Drucksensor 18 wurden auf derselben Seite einer Leiterplatine 6 mit eingebetteter NFC-Antenne 20 verbaut und zwar auf derjenigen Seite, die später zur Innenseite der Kuppel ausgerichtet sein würde. Die derart vorbereitete Leiterplatine 6 wurde mithilfe eines aus Polypropylen bestehenden beidseitig klebenden Klebebands 2 in die Basis 1 eingeklebt, so dass die Sensoren 12, 18 von der Basis 1 wegzeigten. Zwei in der Basis 1 vorintegrierte Platzierungsaussparungen halfen die Leiterplatine 6 korrekt auszurichten. Oberhalb des ersten Drucksensors 12 und der Leiterplatine 6 wurde eine Halterung 16 aus TPE platziert. Hierzu verfügte die Halterung 16 über eine Öffnung 7 für den ersten Drucksensor 12, durch welche der Sensor umschlossen wurde. Die Halterung 16 trennte den ersten Drucksensor 12 vom späteren Gehäuseinnenraum und fungierte somit als gasdichte Barriere zwischen dem ersten Drucksensor 12 und dem zweiten Drucksensor 18. Damit der erste Drucksensor 12 mit dem Außenluftdruck in Kontakt stehen konnte, verfügte die Öffnung 7 über einen lateral, in rechtem Winkel zum Sensor, gelegenen Tunnel als Luftzugang.

Die verwendete Kuppel 3 hatte eine mehrheitlich planare Fläche, die jedoch eine Erhebung für die Halterung auswies. Die Erhebung überlagerte auch den zweiten Drucksensor 18. Die Erhebung war oberhalb der Halterung 16 bogenförmig ausgestaltet und oberhalb des zweiten Drucksensors 18 in lateraler Richtung konisch spitz zulaufend. Auf diese Weise wurde bei der Kuppel 3 Material eingespart. Basis 1, Halterung 16 und Kuppel 3 wurden miteinander verschweißt. Die erhaltene mobile Druckmesseinheit hatte eine maximale Höhe von 3,8 mm. Der Aufbau entsprach der in Fig. 9 dargestellten Anordnung.

### Beispiel 6: Verstärkte mobile Druckmesseinheit in Form eines abgerundeten Quadrats

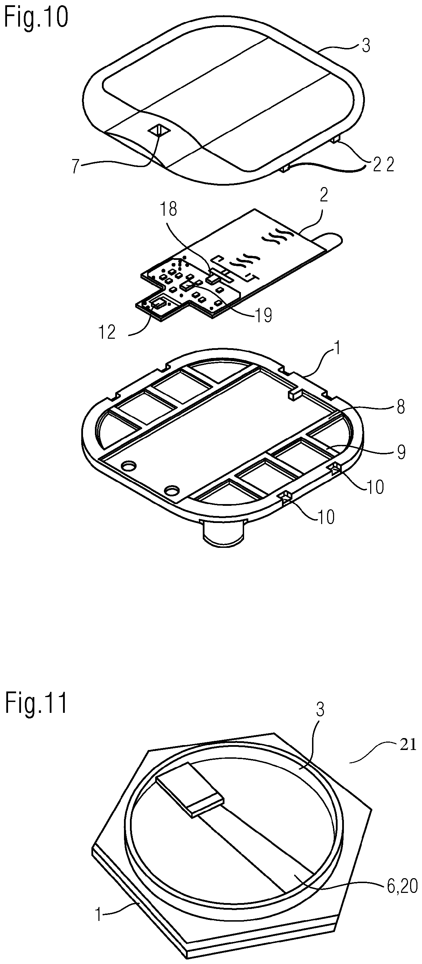

Bereitgestellt wurde eine Basis 1 nach Beispiel 5, allerdings mit kontinuierlichem Rahmen 8 für die Leiterplatine 6. Um den Rahmen 8 herum waren rechtwinklig zum Rahmen Verstärkungsstreben angeordnet. Die Basis 1 hatte im Randbereich insgesamt sechs Aussparungen 10 zur sowohl erleichterten als auch stärkeren Befestigung der Kuppel 3. Dabei waren an drei Seiten der quadratischen Basis 1 jeweils zwei Aussparungen 10. Die vierte Seite besaß aus Platzgründen keine Aussparungen, da zu dieser Seite hin ausreichend Raum für den ersten Drucksensor 7 benötigt wurde. Eine Leiterplatine 6 mit eingebetteter NFC-Antenne 20 und beiden Drucksensoren 12, 18 auf derselben Platinenseite wurde bereitgestellt. Die Platine 6 wurde im Rahmen 8 mithilfe eines beidseitig klebenden Klebebands 2 fixiert, so dass die Sensoren 12, 18 von der Basis 1 wegzeigten. Die Kuppel 3 verfügte über sechs stiftförmige Vorsprünge 22, die bündig in die Aussparungen 10 der Basis 1 eingeführt wurden. Ferner hatte die Kuppel 3 eine Öffnung 7 für den ersten Drucksensor und eine Halterung 16, die den ersten Drucksensor 12 gasdicht vom zweiten Drucksensor 18 und dem Gehäuseinneren trennte. Die aufgesetzte Kuppel 3 wurde gasdicht mit der Basis 1 verschweißt. Der Aufbau dieser Version der mobilen Druckmesseinheit kann Fig. 10 entnommen werden, wobei die Halterung 16 nicht separat ausgewiesen ist, aber als Erhebung um die Öffnung 7 herum erkennbar ist.

Da bei dieser Version der mobilen Druckmesseinheit die Öffnung 7 in der Kuppel liegt 3, sollte die Messeinheit mit der Basis 1 voran auf die Haut aufgelegt werden, damit die Öffnung 7 während der Messungen nicht von der Haut bedeckt ist und der Luftaustausch erhalten bleibt.

### Beispiel 7: Mobile Druckmesseinheit in hexagonaler Form

Es wurde eine sechseckige Basis 1 aus TPE mit jeweils derselben Kantenlänge bereitgestellt. Die Basis 1 hatte eine zentrisch angelegte kreisförmige Vertiefung. Der erhabene Bereich um die Vertiefung bildete den Rand. Ferner verfügte die Basis 1 an ihrer Innenseite über eine durchgehende, bis zur Außenseite reichende Öffnung 7 für den ersten Drucksensor 12. Die Platine 6 mit eingebetteter NFC-Antenne 20 und den beiden Sensoren 12,18 an gegenüberliegenden Seiten der Platine 6 wurde mittels einer viereckigen Halterung 16 aus TPE an der Basis 1 befestigt, und zwar so, dass der erste Drucksensor 12 in der erwähnten Öffnung 7 positioniert wurde. Der zweite Drucksensor 18 wurde von der Halterung 16 überlagert, aber nicht gasdicht umschlossen, so dass er mit dem gasgefüllten Innenraum des Gehäuses 21 in Kontakt stand.

Die derart vorbereitete Basis 1 wurde gasdicht verschweißt mit einer transparenten, ebenfalls hexagonalen Kuppel 3. Die Kuppel 3 hatte ebenfalls einen zentrisch ausgerichteten kreisförmigen Bereich, wobei der kreisförmige Bereich den hexagonalen Randbereich überragte. Diese Ausführung ist bildlich in Fig. 11 dargestellt.

### Kurze Beschreibung der Zeichnungen

Fig. 1 zeigt eine Leiterplatine 6 in der Aufsicht. An der Leiterplatine 6 ist ein erster digitaler Drucksensor 12 befestigt. Der erste Drucksensor 12 liegt vom Betrachter ausgehend unterhalb der Leiterplatine 6 und ist deshalb angedeutet dargestellt. Weiterhin ist an der Leiterplatine 6 ein zweiter digitaler Drucksensor 18 befestigt, der vom Betrachter ausgehend auf der Oberseite der Leiterplatine 6 liegt. In die Leiterplatine 6 eingelassen ist eine NFCkompatible Antenne 20 in Form einer Rahmenantenne. Weitere elektronische Komponenten können je nach Bedarf auf der dargestellten Leiterplatine 6 montiert werden. Die dargestellte Leiterplatine 6 eignet sich, um in der erfindungsgemäßen mobilen Druckmesseinheit eingesetzt zu werden. Sie kann beispielsweise an einer Basis 1 der Druckmesseinheit fixiert werden.

Fig. 2 zeigt eine insgesamt flach ausgebildete Kuppel 3 in der Aufsicht. Die Kuppel 3 ist aus PU gefertigt und kreisförmig. Die Kuppel 3 ist auf ihrer (dem Betrachter zugewandten) Außenseite im kreisförmigen Bereich vollflächig von einer beidseitig mit hautfreundlichem Acrylkleber beschichteten Folie 4 überlagert, welche zum Schutz mit einer Abdeckfolie 5 bedeckt ist. Die Abdeckfolie 5 kann unmittelbar vor dem Anbringen der Kuppel 3 an die Haut über die Lasche 14 (Lasche zum Abziehen der Abdeckfolie) händisch entfernt werden. Nach dem Einsatz kann die Kuppel 3 über die Lasche 15 (Lasche zum Abziehen der mobilen Druckmesseinheit von der Haut des Benutzers) entfernt werden. Die dargestellte Kuppel 3 eignet sich für den Einsatz in der erfindungsgemäßen mobilen Druckmesseinheit. Sie kann beispielsweise mit einer Basis 1 verbunden werden, auf welcher weitere notwendige Komponenten wie die Leiterplatine 6 befestigt sind. Laschen 14, 15 und Klebefolie 4 sind optional und steigern den Bedienungskomfort.

Fig. 3 zeigt ein Gehäuse 21 in der Seitenansicht. Das Gehäuse 21 besteht aus einer Kuppel 3 und einer Basis 1, die gasdicht miteinander verschweißt sind. Auf der Unterseite der Basis 1 befindet sich eine Öffnung 7 zur Befestigung des ersten Drucksensors 12. Die Öffnung 7 liegt als Ausstülpung vor und ragt über die Basis 1 hinaus. Das dargestellte Gehäuse 21 eignet sich, um eine Leiterplatine 6 und weitere Komponenten aufzunehmen. Das Gehäuse 21 kann mit der Kuppel 3 voran auf die Haut aufgelegt werden. Ein in der Öffnung 7 untergebrachter erster Drucksensor 12 bleibt dabei im Austausch mit der atmosphärischen Luft.

Fig. 4 zeigt ein Gehäuse 21. Dem Betrachter zugewandt ist dabei die Basis 1. In die Basis 1 ist eine Öffnung 7 zur Befestigung des ersten Drucksensors eingelassen. Die Öffnung ist derart ausgestaltet, dass der Drucksensor bestmöglich geschützt wird, ohne dass der Kontakt mit der atmosphärischen Luft verloren geht. Die Laschen 14 und 15 steigern den Bedienkomfort.

Fig. 5 zeigt den schichtweisen Aufbau einer erfindungsgemäßen Druckmesseinheit. Die Basis 1 mit Lasche 15 ist mit ihrer Innenseite zum Betrachter dargestellt. Auf dieser Innenseite befindet sich ein Rahmen 8 zum Einsetzen der Leiterplatine 6. Der Rahmen 8 bildet hier eine durchgehende Erhebung auf der Innenseite der Basis 1, so dass die Leiterplatine 6 bündig vom Rahmen 8 umschlossen wird. Die Leiterplatine 6 ist mithilfe eines beidseitig klebenden Klebebands 2 mit der Basis verbunden. Das Klebeband 2 hat die gleich äußeren Abmessungen, wie die Leiterplatine 6, verfügt jedodoch über eine Aussparung für den ersten Drucksensor 12, die den ersten Drucksensor gasdicht umschließt und einen Austritt von Gas aus dem Gehäuse 21 durch die Öffnung 7 verhindert. Der an der Unterseite der Leiterplatine 6 angebrachte erste Drucksensor 12 findet Platz in der Öffnung 7, welche sich ebenfalls innerhalb des Rahmens 8 befindet. Auf der Oberseite der Leiterplatine 6 befindet sich der zweite Drucksensor 18. In die Platine 6 eingelassen ist eine NFCkompatible Antenne 20. Der Detailaufbau der Platine 6 kann den Fig. 1 und 10 entnommen werden.

Die Basis 1 wird überlagert von der Kuppel 3 und ist mit dieser gasdicht verbunden. Die Kuppel 3 ist an ihrer Oberseite mit einer beidseitig klebenden Folie 4 bedeckt. Die Oberseite der Folie 4 ist beschichtet mit hautkompatiblem Acrylkleber, welcher durch eine Abdeckfolie 5 geschützt ist, die über eine Abzugslasche 14 entfernt werden kann. Nach dem Entfernen der Abdeckfolie 5 kann die Kuppel 3 mittels der Klebefolie 4 auf der Haut fixiert. Wird die Druckmesseinheit nicht länger benötigt, kann sie über Lasche 15 abgezogen werden.

Fig. 6 zeigt die Innenseite einer Basis 1 mit Rahmen 8 und Öffnung 7 für den ersten Drucksensor. Die hier dargestellte Version der Basis 1 verfügt über Verstärkungsstreben 9. Die Verstärkungsstreben 9 umgeben den Rahmen 8 und erhöhen die Verwindungssteifigkeit der Basis 1, so dass die einzusetzende Leiterplatine 6 besser vor Biege- und Torsionskräften geschützt ist. Die Basis 1 verfügt zusätzlich über Aussparungen 10 zur leichteren Befestigung der Kuppel 3 auf der Basis 1. Die zugehörige Kuppel 3 weist zu jeder Aussparung 10 einen stiftförmigen Vorsprung 22 auf, der beim Aufsetzen der Kuppel bündig mit der jeweiligen Aussparung 10 abschließt. Dies erleichtert die gasdichte Befestigung der Kuppel 3 an der Basis 1 und stellt sicher, dass die Kuppel 3 beispielsweise während des Schweißvorgangs nicht verrutscht. Zudem wird die Belastbarkeit des resultierenden Gehäuses 21 gegenüber mechanischen Kräften erhöht.

Fig. 7 zeigt eine andere Variante der Basis 1 in der Innenansicht. Der Rahmen 8 für die Leiterplatine 6 ist diskontinuierlich ausgestaltet und besteht aus sechs Erhebungen, die allesamt mit der einzusetzenden Leiterplatine 6 bündig abschließen, so dass ein Verrutschen der Leiterplatine 6 verhindert wird. Der diskontinuierliche Rahmen 8 spart im Vergleich zu einem kontinuierlichen Rahmen Material. Zudem wird das Gewicht der Basis 1 und somit der Druckmesseinheit insgesamt reduziert, so dass sie sich leichter an der Haut fixieren lässt.

Die Öffnung 7 zur Befestigung des ersten Drucksensors ist umgeben von mehreren Einkerbungen 11 für das Einfüllen von Epoxidharz 13. Das Epoxidharz 13 wird nach dem Einsetzen des ersten Drucksensors in die Einkerbungen 11 gefüllt und der Sensor auf diese Weise in der Öffnung 7 fixiert. Anschließend kann die Leiterplatine in den Rahmen 8 eingesetzt und mit dem Sensor 7 verbunden werden.

Fig. 8 zeigt dieselbe Variante der Basis 1 mit eingesetztem ersten Drucksensor 12 und dem Einbringen von Epoxidharz 13.

Fig. 9 zeigt eine andere Variante der mobilen Druckmesseinheit im Schichtaufbau. Bei dieser Ausführungsform ist die Druckmesseinheit in der Aufsicht als Quadrat mit abgerundeten Ecken ausgestaltet. Hierdurch entsteht eine größere Auflagefläche zur Haut, die dem Verrutschen der Druckmesseinheit besonders effektiv entgegenwirkt. Diese Variante eignet sich besonders gut für behaarte Haut an weniger beweglichen Körperbereichen. Die Basis 1 ist an ihrer Außenseite mit einer beidseitig klebenden Folie 4 versehen, die durch eine Abdeckfolie 5 geschützt ist. Die Abdeckfolie 5 verfügt über eine Lasche 14. Über diese Lasche 14 kann die Abdeckfolie 5 unmittelbar vor dem Ankleben der mobilen Druckmesseinheit auf die Haut abgezogen werden. Die Druckmesseinheit wird mit der Basis 1 voran an die Haut angebracht. Die Leiterplatine 6 ist mithilfe eines doppelseitig klebenden Klebebands 2 in der Basis 1 befestigt. Auf die Leiterplatine 6 aufgesteckt sind der erste Drucksensor 12 und der zweite Drucksensor 18. Beide Drucksensoren 12, 18 befinden sich auf derselben Seite des Leiterplatine 6 und zeigen zum Betrachter. Der erste Drucksensor 12 wird von einer Halterung 16 überlagert und auf diese Weise räumlich vom zweiten Drucksensor 18 getrennt. Die Halterung 16 hat eine Öffnung 7, über welche der erste Drucksensor 12 mit der atmosphärischen Luft in Kontakt steht.

Die Basis 1 ist verschweißt mit einer Kuppel 3, welche über eine zusätzliche Erhebung verfügt, die oberhalb der Haltung 16 liegt und einen Luftaustausch mit dem ersten Drucksensor 12 über die Öffnung 7 sicherstellt.

Fig. 10 zeigt eine weitere Variante der mobilen Druckmesseinheit. Basis 1 und Kuppel 3 haben in der Aufsicht die Form eines Quadrats mit abgerundeten Ecken. Auf der Leiterplatine 6 sind der erste Drucksensor 12 und der zweite Drucksensor 18 aufgesteckt. Die Leiterplatine 6 enthält darüber hinaus einen Mikrocontroller 19. Zwischen der Basis 1 und der Leiterplatine 6 befindet sich ein beidseitig klebendes Klebeband 2 (in der Abbildung an der Unterseite der Leiterplatine 6), welches die Leiterplatine 6 an der Basis 1 fixiert. Die Kuppel 3 verfügt Stifte 22, die bündig mit den Aussparungen 10 der Basis 1 abschließen. Der erste Drucksensor 12 befindet sich in einer Öffnung 7 der Kuppel 3 und steht über diese mit der atmosphärischen Luft in Kontakt, wohingegen der zweite Drucksensor 18 lediglich mit dem Gas innerhalb des gasdichten Gehäuses 21 in Kontakt steht.

Fig. 11 zeigt eine weitere Variante der mobilen Druckmesseinheit, bei welcher die Basis 1 in der Aufsicht ein Hexagon mit gleichlangen Seiten bildet. Die Kuppel 3 ist transparent gestaltet, so dass innerhalb des Gehäuses 21 die Leiterplatine 6 mit integrierter Antenne 20 sichtbar ist.

## Patentansprüche

1. Mobile Druckmesseinheit zur Messung des Anpressdrucks, welcher von einer medizinischen Kompressionsbinde auf eine Extremität eines Patienten ausgeübt wird, umfassend
a) ein gasdichtes Gehäuse 21, das eine flexible Kuppel 3 sowie in seinem Inneren einen gasgefüllten Hohlraum aufweist, um den Anpressdruck der über dem Gehäuse angelegten Kompressionsbinde aufzunehmen,
b) einen ersten Drucksensor 12, welcher mit der atmosphärischen Luft kommuniziert, zur Messung des Außen-Luftdrucks, wobei der Drucksensor 12 an der Gehäuseaußenseite angebracht ist oder zumindest teilweise in das Gehäuse eingelassen ist,
c) einen zweiten Drucksensor 18, welcher innerhalb des gasgefüllten Hohlraums vorhanden ist und welcher mit diesem kommuniziert, zur Messung des Innen-Gasdrucks in dem gasgefüllten Hohlraum,
d) eine Leiterplatine 6, welche in dem gasgefüllten Hohlraum vorhanden ist und einen passiven RFID-Transponder umfasst,
wobei die Leiterplatine 6 mit dem ersten Drucksensor 12 und dem zweiten Drucksensor 18 elektrisch verbunden ist,
und wobei unter Einsatz eines RFID-Lesegerätes Druckmessungen durch die Drucksensoren durchgeführt und die so ermittelten Daten kabellos ausgelesen werden können.

2. Mobile Druckmesseinheit nach Anspruch 1, wobei das Gehäuse eine Basis 1 aufweist, die der flexiblen Kuppel 3 gegenüberliegt und die eine der Kuppel 3 zugewandte Innenseite sowie eine der Innenseite gegenüberliegende Außenseite hat, wobei die Basis 1 und die flexible Kuppel 3 gasdicht miteinander verbunden sind, so dass zwischen Basis 1 und flexiblem Bereich 3 der gasgefüllte Hohlraum ausgebildet wird.

3. Mobile Druckmesseinheit nach Anspruch 2, wobei die Basis 1 gummielastisch ist, und wobei die Basis einen Shore-A-Wert von 20 bis 80 aufweist.

4. Mobile Druckmesseinheit nach Anspruch 2 oder 3, wobei die Basis 1 Silikon oder ein thermoplastisches Polyurethan enthält oder damit beschichtet ist.

5. Mobile Druckmesseinheit nach einem der Ansprüche 2 bis 4, wobei die Basis 1 eine Öffnung 7 enthält, in welcher der erste Drucksensor 12 befestigt ist.

6. Mobile Druckmesseinheit nach Anspruch 5, wobei die Öffnung 7 sich in einer nach außen gerichteten Ausstülpung der Basis 1 befindet.

7. Mobile Druckmesseinheit nach Anspruch 5 oder 6, wobei der erste Drucksensor 12 durch ein Epoxidharz 13 oder ein Klebeband 2 befestigt ist.

8. Mobile Druckmesseinheit nach einem der Ansprüche 2 bis 7, wobei die Leiterplatine 6 an der Innenseite der Basis 1 befestigt ist und die Befestigung ein Klebeband 2 umfasst.

9. Mobile Druckmesseinheit nach einem der Ansprüche 2 bis 8, wobei die Innenseite der Basis 1 einen kontinuierlichen oder diskontinuierlichen Rahmen 8 für die Leiterplatine 6 umfasst.

10. Mobile Druckmesseinheit nach einem der Ansprüche 3 bis 9, wobei die Leiterplatine 6 flexibel ist und einen Shore-A-Wert kleiner oder gleich dem Shore-A-Wert der Basis 1 hat.

11. Mobile Druckmesseinheit nach Anspruch 10, wobei die Leiterplatine 6 mindestens ein Polyimid enthält.

12. Mobile Druckmesseinheit nach einem der vorhergehenden Ansprüche, wobei die mobile Druckmesseinheit keine außerhalb des Gehäuses 21 liegenden Kabel und / oder keinen Lagesensor aufweist.

13. Mobile Druckmesseinheit nach einem der vorhergehenden Ansprüche, wobei die mobile Druckmesseinheit keinen Speicher für elektrische Energie enthält.

14. Mobile Druckmesseinheit nach einem der vorhergehenden Ansprüche, wobei die flexible Kuppel 3 aus einer einzigen Schicht von im Wesentlichen einheitlicher Dicke besteht.

15. Verfahren zur Herstellung einer mobilen Druckmesseinheit nach Anspruch 2, umfassend die folgenden Schritte:
a) Bereitstellen der flexiblen Kuppel, der Basis, des ersten und des zweiten, Drucksensors, des RFID-Transponders und der Leiterplatine,
b) Befestigung der Leiterplatine an der Basis,
c) Anschließen der Drucksensoren und des RFID-Transponders an die Leiterplatine, wobei der erste Drucksensor eine Verbindung zur Außenseite der Basis hat
d) Verschweißen der Kuppel mit der Basis, so dass zwischen der Kuppel und der Basis ein gasdichter gasgefüllter Hohlraum ausgebildet wird und so dass Kuppel und Basis den zweiten Drucksensor, den Transponder und die Leiterplatine umschließen.
